# EUROPÄISCHE PATENTSCHRIFT

(11) **EP 0 970 057 B1**
(45) Veröffentlichungstag und Bekanntmachung des Hinweises auf die Patenterteilung: **01.02.2006**
(21) Anmeldenummer: 98913618.9
(22) Anmeldetag: 02.03.1998
(51) Int. Cl.: C07D 239/52

(54) **SUBSTITUIERTE AMINOSALICYLSÄUREAMIDE MIT FUNGIZIDER WIRKUNG UND ZWISCHENPRODUKTE ZU IHRER HERSTELLUNG**
SUBSTITUTED AMINOSALICYCLIC ACID AMIDES WITH FUNGICIDAL EFFECT AND INTERMEDIATE PRODUCTS FOR PRODUCTION THEREOF
AMIDES D'ACIDE AMINOSALICYLIQUE SUBSTITUES A EFFET FONGICIDE ET PRODUITS INTERMEDIAIRES POUR LEUR PREPARATION

(30) Priorität: 14.03.1997 DE 19710609
(43) Veröffentlichungstag der Anmeldung: 12.01.2000
(60) Teilanmeldung: 05002366.2
(73) Patentinhaber: Bayer CropScience AG, 40789 Monheim (DE)
(72) Erfinder: SEITZ, Thomas, D-40764 Langenfeld (DE); STELZER, Uwe, D-51399 Burscheid (DE); WOLFRUM, Peter, D-40789 Monheim (DE); STENZEL, Klaus, D-40595 Düsseldorf (DE)
(74) Vertreter: Weiss, Wolfgang
(86) Internationale Anmeldenummer: PCT/EP1998/001164
(87) Internationale Veröffentlichungsnummer: WO 1998/041513

(56) Entgegenhaltungen:
- EP-A- 0 382 375
- EP-A- 0 463 488
- WO-A-92/18487
- WO-A-94/02470
- WO-A-95/14674
- WO-A-95/24383
- WO-A-97/08135
- DE-A- 2 120 861
- SELWOOD,D.L. ET AL.: "Structure-Activity Relationships of Antifilarial Antimycin Analogues: A Multivariate Pattern Recognition Study" J.MED.CHEM., Bd. 33, Nr. 1, 1990, Seiten 136-142, XP002071789 WASHINGTON in der Anmeldung erwähnt
- TOKUTAKE,N. ET AL.: "Inhibition of electron transport of rat-liver mitochondria by synthesized antimycin A analogs" BIOCHIM.BIOPHYS.ACTA, Bd. 1142, 1993, Seiten 262-268, XP002071909
- SO,S.-S. ET AL.: "Evolutionary Optimization in Quantitative Structure-Activity Relationship: An Application of Genetic Neural Networks" J.MED.CHEM., Bd. 39, Nr. 7, 1996, Seiten 1521-1530, XP002071790 WASHINGTON

## Beschreibung

Die Erfindung betrifft neue substituierte Aminosalicylsäureamide, mehrere Verfahren zu ihrer Herstellung und ihre Verwendung als Fungizide, sowie neue Zwischenprodukte und mehrere Verfahren zu deren Herstellung.

Bestimmte substituierte Acylaminosalicylsäureamide, wie beispielsweise die Verbindungen 3-Formamido-salicylanilid und 3-(Formylamino)-2-hydroxy-N-(phenylmethyl)-benzamid, sind bereits bekannt (vergleiche z. B. Biochim. Biophys. Acta (1993), 1142(3), 262-8, J. Med. Chem. (1990), 33(1), 136-42 oder J. Biol. Chem. (1971), 246(23), 7125-30). Eine Wirkung gegen Schädlinge ist von diesen vorbekannten Verbindungen bisher jedoch nicht beschrieben.

Es wurden nun die neuen substituierten Aminosalicylsäureamide der allgemeinen Formel (I) gefunden, 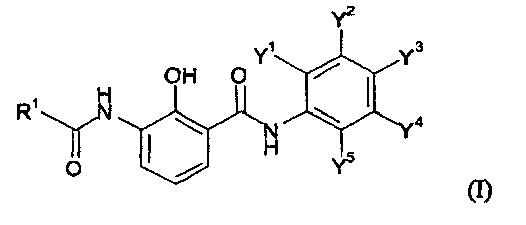
in welcher
- R¹: für Wasserstoff, Methyl oder Methoxy steht.
- Y¹, Y², Y³, Y⁴ und Y⁵: gleich oder verschieden sind und für Wasserstoff, Fluor, Chlor, Brom, Cyano, Methyl, Ethyl, Methoxy, Trifluormethyl oder Difluormethoxy stehen.
- Y² oder Y³: für -G-Z steht,
worin
G für -T-Ar¹- oder -T-Ar¹-O- steht,
worin
Ar¹ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff Schwefel, -CH₂-O-, CH₂-S-, Methylen, Ethylen oder Propylen steht.
Z für gegebenenfalls einfach bis dreifach, gleich oder verschieden durch Fluor, Chlor, Cyano oder Methoxy substituiertes Methyl, Ethyl, n- oder i-Propyl, n-, i-, s- oder t-Butyl;
für jeweils gegebenenfalls durch Fluor oder Chlor substituiertes Vinyl, Allyl oder Propargyl;
für jeweils gegebenenfalls einfach bis dreifach, gleich oder verschieden durch Fluor, Chlor, Cyano, Methoxy, Phenyl, Methyl oder Ethyl substituiertes Cyclopropyl, Cyclobutyl, Cyclopentyl oder Cyclohexyl steht; oder
Z für jeweils gegebenenfalls einfach bis dreifach, gleich oder verschieden substituiertes Phenyl, Pyridyl, Pyrimidyl oder Thienyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Cyclopropyl, Cyclobutyl, Cyclopentyl, Cyclohexyl, Phenyl, Phenoxy, oder
eine Gruppierung 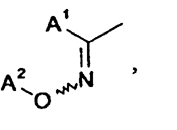
worin
   A¹ für Methyl, Ethyl, n- oder i-Propyl, n-, i-, s- oder t-Butyl, Cyclopropyl oder Cyclobutyl steht,
   A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl; Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 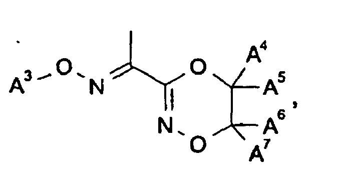
in welcher
   A³ für Methyl oder Ethyl steht und
   A⁴, A⁵, A⁶ und A⁷ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
   A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoffatomen bilden.

In den Definitionen sind die Kohlenwasserstoffketten, wie Alkyl, Alkylen, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Weiterhin wurde gefunden, daß man die neuen substituierten Acylaminosalicylsäureamide der allgemeinen Formel (I) erhält, wenn man
a) Aminosalicylsäureamide der allgemeinen Formel (II), 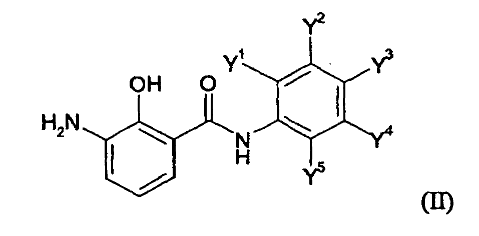
   in welcher
   Y¹, Y², Y³, Y⁴ und Y⁵ die oben angegebenen Bedeutungen haben,
   mit Acylierungsmitteln der allgemeinen Formel (III), 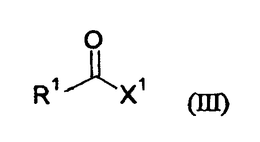
   in welcher
   - R¹: die oben angegebene Bedeutung hat und
   - X¹: für Halogen, Hydroxy, Alkoxy oder Alkylcarbonyloxy steht,

   gegebenenfalls in Gegenwart eines Verdünnungsmittels, gegebenenfalls in Gegenwart eines Säureakzeptors, und gegebenenfalls in Gegenwart eines weiteren Reaktionshilfsmittels, umsetzt, oder wenn man
b) Nitrosalicylsäureamide der allgemeinen Formel (IV) 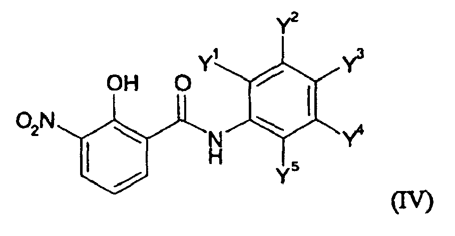
   in welcher
   Y¹, Y², Y³, Y⁴ und Y⁵ die oben angegebenen Bedeutungen haben,
   mit Ameisensäure, gegebenenfalls in Gegenwart eines Katalysators und gegebenenfalls in Gegenwart eines weiteren Reaktionshilfsmittels, umsetzt, oder wenn man
c) O-Benzyl-nitrosalicylsäureamide der allgemeinen Formel (V), 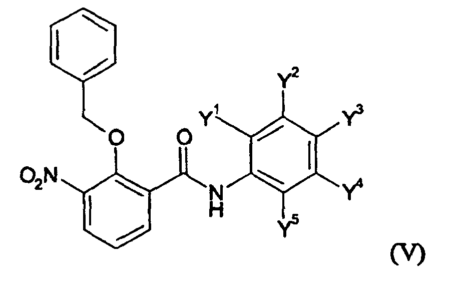
   in welcher
   Y¹, Y², Y³, Y⁴ und Y⁵ die oben angegebenen Bedeutungen haben,
   mit Ameisensäure, gegebenenfalls in Gegenwart von Wasserstoff oder eines unedlen Metalls, gegebenenfalls in Gegenwart eines Katalysators und gegebenenfalls in Gegenwart eines weiteren Reaktionshilfsmittels, umsetzt.

Schließlich wurde gefunden, daß die neuen substituierten Aminosalicylsäureamide der allgemeinen Formel (I) eine sehr starke fungizide Wirkung zeigen.

Die erfindungsgemäßen Verbindungen und deren Vorprodukte liegen gegebenenfalls als Mischungen verschiedener möglicher isomerer Formen, insbesondere von Stereoisomeren, wie z. B. E- und Z-, threo- und erythro-, sowie optischen Isomeren oder auch Tautomeren vor. Es werden sowohl die E- als auch die Z-Isomeren, wie auch die threo- und erythro-, sowie die optischen Isomeren, als auch die möglichen Tautomeren, sowie beliebige Mischungen dieser Isomeren beansprucht.

In der allgemeinen Formel (I) steht
- R1: besonders bevorzugt für Wasserstoff.

In der allgemeinen Formel (I) stehen
Y¹,Y⁴ und Y⁵ unabhängig voneinander bevorzugt für Wasserstoff.

Insbesondere steht Y² für Wasserstoff, Fluor, Chlor, Brom, Cyano, Methyl, Ethyl, Methoxy, Trifluormethyl oder Difluormethoxy; bevorzugt für Wasserstoff.

Insbesondere steht Y³ für Wasserstoff, Fluor, Chlor, Brom, Cyano, Methyl, Ethyl, Methoxy, Trifluormethyl oder Difluormethoxy; bevorzugt für Gruppe -G-Z.

Bevorzugt steht G für -T-Ar¹ und insbesondere für -T-Ar¹-O-, wobei
- T: insbesondere für eine Einfachbindung, für Sauerstoff, Schwefel, -CH₂-O-, -CH₂-S-, Methylen, Ethylen oder Propylen und bevorzugt für Sauerstoff steht.
- Ar¹: steht bevorzugt
für 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden substituiertes Pyridindiyl, Pyrimidindiyl oder 1,3,5-Triazindiyl, wobei die die möglichen Substituenten vorzugsweise aus der nachstehenden Aufzählung ausgewählt sind: Fluor, Chlor, Brom, Cyano, Methyl, Cyclopropyl, Methoxy, Methylthio, Trifluormethyl, Difluormethoxy, Trifluormethoxy, Difluorchlormethoxy, oder besonders bevorzugt für 1,2,4-Thiadiazoldiyl oder gegebenenfalls einfach oder zweifach, gleich oder verschieden substituiertes Pyrimidindiyl steht, wobei die oben angegebenen Substituenten in Frage
kommen und die Substituenten vorzugsweise für Halogen, insbesondere für Fluor stehen.

In der allgemeinen Formel (I) steht
- Z: bevorzugt für gegebenenfalls einfach bis dreifach, gleich oder verschieden durch Fluor, Chlor, Cyano oder Methoxy substituiertes Methyl, Ethyl, n- oder i-Propyl, n-, i-, S- oder t-Butyl;
für jeweils gegebenenfalls durch Fluor oder Chlor substituiertes Vinyl, Allyl oder Propargyl;
für jeweils gegebenenfalls einfach bis dreifach, gleich oder verschieden durch Fluor, Chlor, Cyano, Methoxy, Phenyl, Methyl oder Ethyl substituiertes Cyclopropyl, Cyclobutyl, Cyclopentyl oder Cyclohexyl;
und bevorzugt für jeweils gegebenenfalls einfach bis dreifach, gleich oder verschieden substituiertes Phenyl, Pyridyl, Pyrimidyl oder Thienyl, wobei die möglichen Substituenten die oben angegebenen Bedeutungen haben und vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Dißuorchlormethylihio, Trifluormethylsulfinyl, Trifluormethylsulfonyl, Methoxycarbonyl, Ethoxycarbonyl oder Phenoxy;
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy;
Cyclopropyl, Cyclobutyl, Cyclopentyl, Cyclohexyl, Phenyl, Phenoxy oder
eine Gruppierung 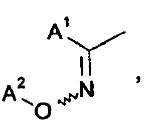
worin
   A¹ für Methyl, Ethyl, n- oder i-Propyl, n-, i-, s- oder t-Butyl, Cyclopropyl oder Cyclobutyl steht,
   A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 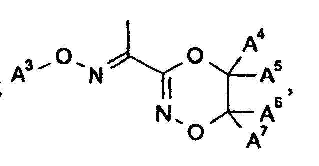
in welcher
   A³ für Methyl oder Ethyl steht und
   A⁴, A⁵, A⁶ und A⁷ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
   A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoffatomen bilden.

In der allgemeinen Formel (I) steht
- Z: besonders bevorzugt für jeweils gegebenenfalls einfach bis dreifach, gleich oder verschieden substituiertes Pyridyl, Pyrimidyl und insbesondere für unsubstituiertes oder einfach bis dreifach, gleich oder verschieden substituiertes Phenyl, wobei die möglichen Substituenten die oben angegebenen Bedeutungen haben und vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trißuormethoxy, Difluorchlormethoxy, Trifluorethoxy, Difluormethylthio, Trifluormethylthio, Difluorchlormethylthio, Trifluormethylsulfinyl, Trifluormethylsulfonyl, Methoxycarbonyl, Ethoxycarbonyl oder Phenoxy.

Die oben aufgeführten allgemeinen oder in den Vorzugsbereichen angegebenen Reste-definitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Rest angegebenen Restedefinitionen werden, unabhängig von der jeweilig angegebenen Kombination, beliebig durch entsprechende Restedefinition aus anderen Vorzugsbereichen ersetzt.

Beispielhaft und vorzugsweise werden in den Tabellen 2 bis 4 erfindungsgemäße Verbindungen aulgeführt:

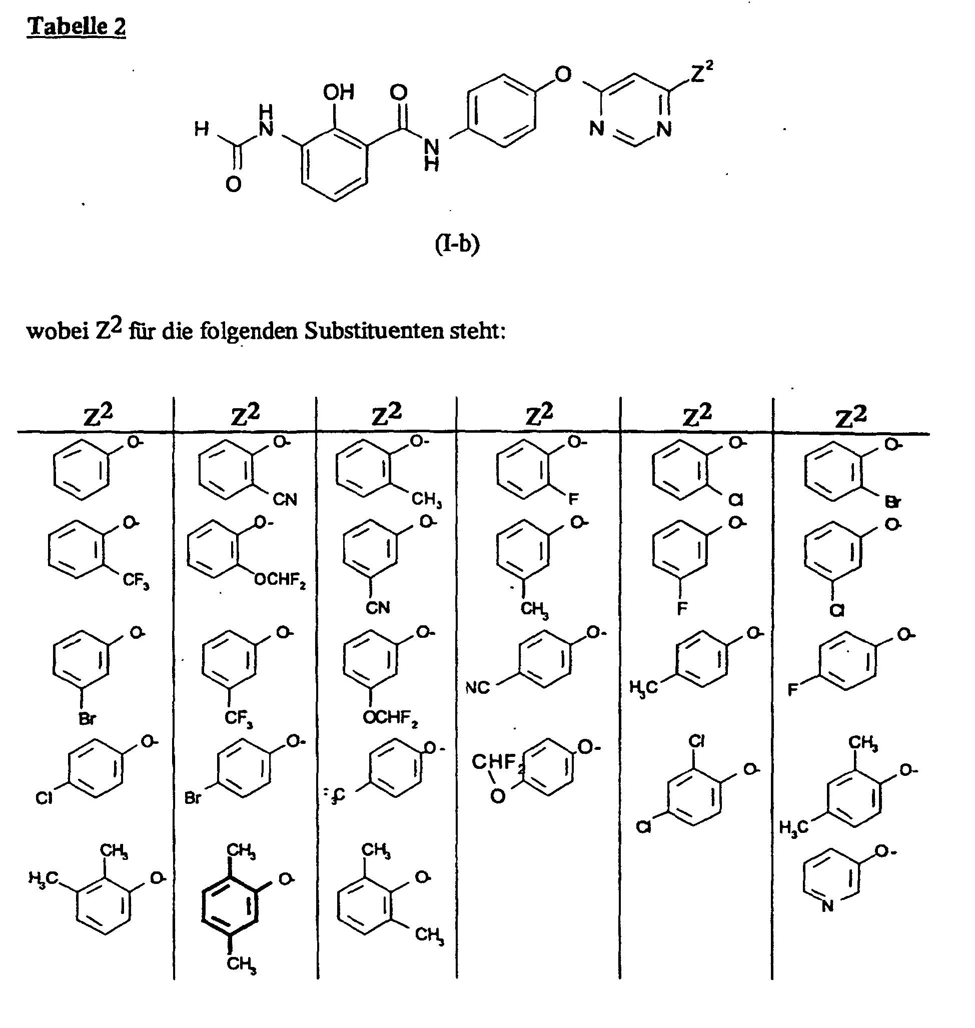

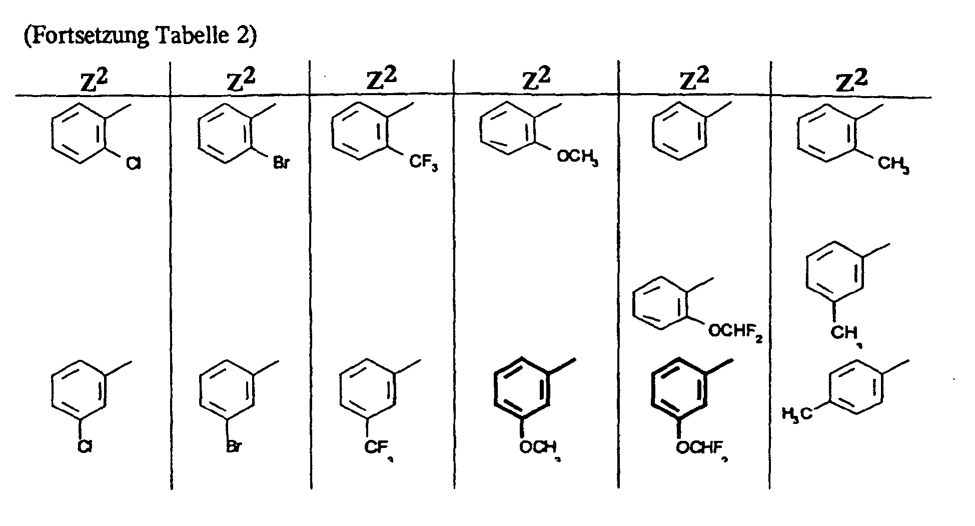

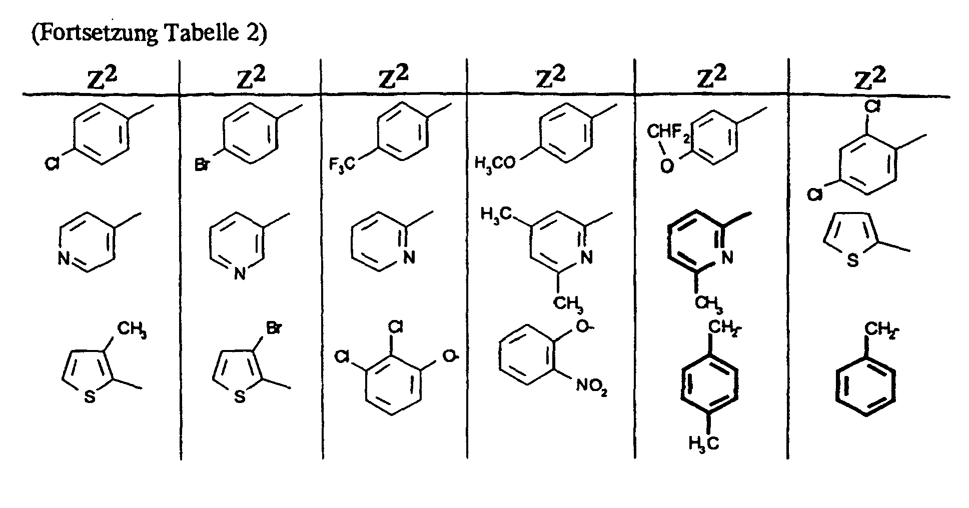

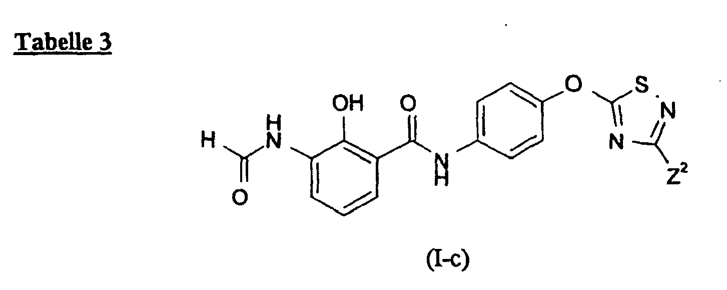

wobei Z² für die in Tabelle 2 genannten Substituenten steht.

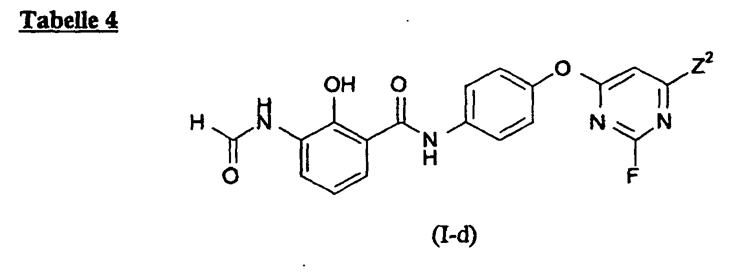

wobei Z² für die in Tabelle 2 genannten Substituenten steht.

Die zur Durchführung des erfindungsgemäßen Verfahrens a) als Ausgangsstoffe benötigten Aminosalicylsäureamide sind durch die Formel (II) allgemein definiert. In dieser Formel (II) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die Ausgangsstoffe der Formel (II) sind neu und ebenfalls Gegenstand der vorliegenden Anmeldung.

Die Aminosalicylsäureamide der Formel (II) werden erhalten, wenn man (Verfahren d) Nitrosalicylsäureamide der allgemeinen Formel (IV), 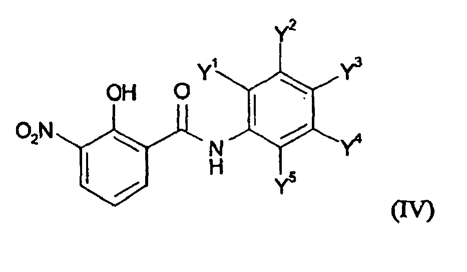
in welcher
Y¹, Y², Y³, Y⁴ und Y⁵ die oben angegebenen Bedeutungen haben,
mit einem Reduktionsmittel, wie beispielsweise Wasserstoff, Eisen, Zink, Zinn-IIchlorid, Natrium- oder Ammoniumhydrogensulfid, gegebenenfalls in Gegenwart eines Verdünnungsmittels und gegebenenfalls in Gegenwart eines Katalysators, umsetzt,
oder wenn man (Verfahren e) O-Benzyl-nitrosalicylsäureamide der allgemeinen Formel (V), 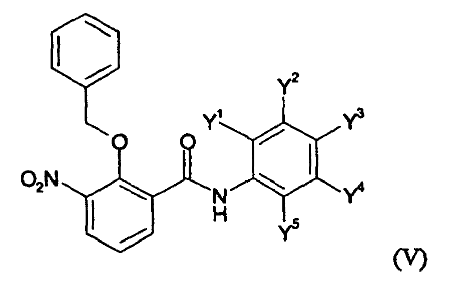
in welcher
Y¹, Y², Y³, Y⁴ und Y⁵ die oben angegebenen Bedeutungen haben,
mit Wasserstoff, gegebenenfalls in Gegenwart eines Verdünnungsmittels, und gegebenenfalls in Gegenwart eines Katalysators, umsetzt.

Die zur Durchführung des erfindungsgemäßen Verfahrens d) als Ausgangsstoffe benötigten Nitrosalicylsäureamide sind durch die Formel (IV) allgemein definiert. In dieser Formel (IV) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die Nitrosalicylsäureamide der Formel (IV) sind neu, und auch Gegenstand der vorliegenden Anmeldung.

Sie werden erhalten, wenn man (Verfahren f) 2-Hydroxy-3-nitrobenzoesäure oder 2-Hydroxy-3-nitrobenzoylchlorid mit einem Amin der Formel (VII), 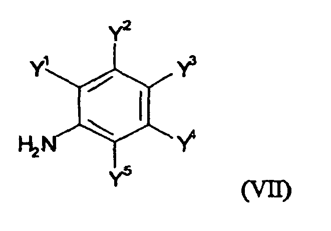
in welcher
Y¹, Y², Y³, Y⁴ und Y⁵ die oben angegebenen Bedeutungen haben,
gegebenenfalls in Gegenwart eines Verdünnungsmittels, gegebenenfalls in Gegenwart eines Kondensationsmittel und gegebenenfalls in Gegenwart eines Säureakzeptors, umsetzt.

Ferner wurde gefunden, daß die neuen Nitrosalicylsäureamide der Formel (IV) sich zur Bekämpfung von Schädlingen an Pflanzen und technischen Materialien, vorzugsweise Pilzen, Insekten und Bakterien, eignen.

Die zur Durchführung des erfindungsgemäßen Verfahrens f) als Ausgangsstoffe benötigte 2-Hydroxy-3-nitrobenzoesäure oder 2-Hydroxy-3-nitrobenzoesäurechlorid sind bekannt (vergleiche z. B. J.Chem. Soc., 1953 2049, 2050 oder US-Patent 03527865).

Die zur Durchführung des erfindungsgemäßen Verfahrens f) weiterhin als Ausgangsstoffe benötigten Amine sind durch die Formel (VII) allgemein definiert. In dieser Formel (VII) haben Y¹, Y², Y³, Y⁴ und Y⁵ vorzugsweise bzw. insbesondere diejenigen Bedeutungen, die bereits im Zusammenhang mit der Beschreibung der erfindungsgemäBen Verbindungen der Formel (I) als bevorzugt bzw. als insbesondere bevorzugt für Y¹, Y², Y³, Y⁴ und Y⁵ angegeben wurden.

Die Amine der Formel (VII) sind teilweise bekannt und können nach bekannten Verfahren hergestellt werden (vergleiche z. B. WO-A 9601825, EP-A 192180).

Neu, und auch Gegenstand der vorliegenden Anmeldung, sind Amine der Formel 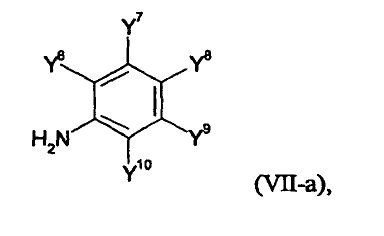
in welcher
- Y⁶, Y⁷, Y⁸, Y⁹ und Y¹⁰: gleich oder verschieden sind und für Wasserstoff, Fluor, Chlor, Brom, Cyano, Methyl, Ethyl, Methoxy, Trifluormethyl oder Difluormethooy stehen, und entweder
- Y⁷ oder Y⁸: für eine Gruppierung 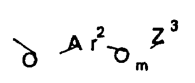 steht,
worin
m für 0 oder 1 steht,
Ar² für 1,2,4-Oxadiazol-3,5-diyl, 1,2,4-Thiadiazol-3,5-diyl oder für gegebenenfalls in 5-Stellung durch Fluor oder Chlor substituiertes Pyrimidin-4,6-diyl steht und
Z³ für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Phenyl, 4-Chlorphenyl, 4-Methylphenyl, Phenoxy, 4-Chlorphenoxy, 4-Methylphenoxy, Cyclopropyl, Cyclobutyl, Cyclopentyl oder Cyclohexyl, oder
eine Gruppierung 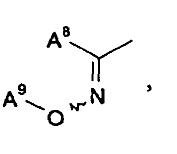
worin
   A⁸ für Methyl, Ethyl, n- oder i-Propyl, n-, i-, s- oder t-Butyl, Cyclopropyl oder Cyclobutyl steht,
   A⁹ für Methyl, Ethyl, n- oder i-Propyl, n-, i-, s- oder t-Butyl, Allyl, Propargyl, But-2-en-1-yl, 2-Methyl-prop-l-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 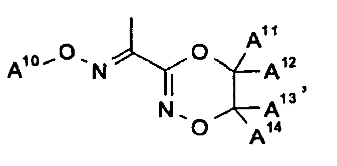
in welcher
   A¹⁰ für Methyl oder Ethyl steht und
   A¹¹, A¹², A¹³ und A¹⁴ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
   A¹¹ und A¹² oder A¹¹ und A¹³ oder A¹³ und A¹⁴ gemeinsam mit den jeweiligen Kohlenstoffatomen, an die sie gebunden sind, einen cycloaliphatischen Ring mit fünf, sechs oder sieben Kohlenstoffatomen bilden.

Die Amine der Formel (VII-a) werden erhalten (Verfahren h), wenn man Diazole der allgemeinen Formel (VIII) oder Halogenpyrimidine der allgemeinen Formel (IX), 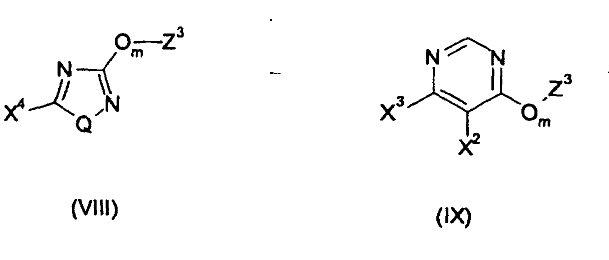
worin
- m: für 0 oder 1 steht,
- X²: für Wasserstoff Fluor oder Chlor steht,
- X³: für Chlor oder Fluor steht,
- X⁴: für Methylsulfonyl, Chlor oder Brom steht und
- Q: für Sauerstoff oder Schwefel steht, und
- Z³: die oben angegebene Bedeutung hat,

mit einem Aminophenol der Formel, 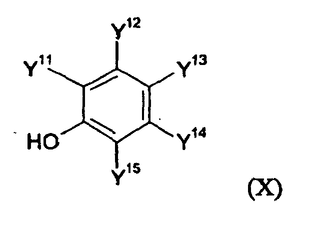
in welcher
Y¹¹, Y¹², Y¹³, Y¹⁴ und Y¹⁵ gleich oder verschieden sind und für Wasserstoff, Halogen, Cyano, Alkyl, Halogenalkyl, Alkoxy oder Halogenalkoxy stehen, und entweder
Y¹² oder Y¹³ für Amino steht,
gegebenenfalls in Gegenwart eines Verdünnungsmittels, gegebenenfalls in Gegenwart eines Säureakzeptors und gegebenenfalls in Gegenwart eines Katalysators, umsetzt, oder wenn man (Verfahren i) Aminophenoxypyrimidine der allgemeinen Formel (XI), 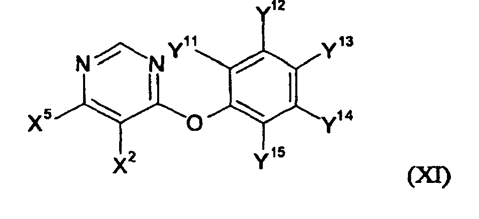
in welcher

- X², Y¹¹, Y¹², Y¹³, Y¹⁴ und Y¹⁵: die oben angegebenen Bedeutungen haben und
- X⁵: für Chlor oder Fluor steht,

mit einem Phenol der allgemeinen Formel (XII),

Z³-OH (XII)

in welcher
- Z³: die oben angegebene Bedeutung hat,

gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens h) als Ausgangsstoffe benötigten Diazole sind durch die Formel (VIII), allgemein definiert. In dieser Formel (VIII) haben m und Z³ vorzugsweise bzw. insbesondere diejenigen Bedeutungen, die bereits im Zusammenhang mit der Beschreibung der erfindungsgemäßen Verbindungen der Formel (VII-a) als bevorzugt bzw. als insbesondere bevorzugt für m und Z³ angegeben wurden. X⁴ steht für Methylsulfonyl; Chlor oder Brom.

Die Diazole der Formel (VIII) sind bekannt und können nach bekannten Methoden hergestellt werden (vergleiche z. B. DE-A 2142913).

Die zur Durchführung des erfindungsgemäßen Verfahrens h) als Ausgangsstoffe alternativ benötigten Halogenpyrimidine sind durch die Formel (IX), allgemein definiert. In dieser Formel (IX) haben m und Z³ vorzugsweise bzw. insbesondere diejenigen Bedeutungen, die bereits im Zusammenhang mit der Beschreibung der erfindungsgemäßen Verbindungen der Formel (VII-a) als bevorzugt bzw. als insbesondere bevorzugt für m und Z³ angegeben wurden. X² steht für Wasserstoff, Fluor oder Chlor. X³ steht für Fluor oder Chlor.

Die Halogenpyrimidine der Formel (IX) sind teilweise bekannt und können nach bekannten Methoden hergestellt werden (vgl. z.B. Pharm.Chem.J.(Engl.Transl.), 23, 6, 1989, 500-503; RU, 23, 6, 1989, 705-707).

Neu, und auch Gegenstand der vorliegenden Anmeldung, sind 4-Chlor-5-fluor-6-phenoxypyrimidine der allgemeinen Formel 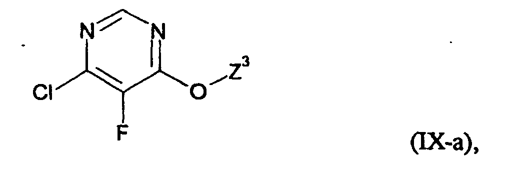
in welcher
- Z³: oben angegebene Bedeutung hat, wobei die Verbindung 2-[(6-Chlor-5-fluor-4-pyrimidinyl)-oxy]-9-(methoxymethylen)-benzoesäuremethylester, ausgenommen ist.

Die neuen 4-Chlor-5-fluor-6-phenoxypyrimidine werden erhalten, wenn man (Verfahren j) Phenole der allgemeinen Formel (XII),

Z³-OH (XII)

in welcher
- Z³: die oben angegebene Bedeutung hat,

mit 4,6-Dichlor-5-fluorpyrimidin (XIII), gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäßen Verfahrens j) als Ausgangsstoffe benötigten Phenole sind durch die Formel (XII) allgemein definiert. In dieser Formel (XII) hat Z³ vorzugsweise bzw. insbesondere diejenige Bedeutung, die bereits im Zusammenhang mit der Beschreibung der erfindungsgemäßen Verbindungen der Formel (VII-a) als bevorzugt bzw. als insbesondere bevorzugt für Z³ angegeben wurde.

Die Phenole der Formel (XII) sind bekannte Synthesechemikalien oder können nach bekannten Verfahren hergestellt werden (WO 95-04728).

Das zur Durchführung des erfindungsgemäßen Verfahrens j) als Ausgangsstoff weiterhin benötigte 4,6-Dichlor-5-fluorpyrimidin (XIII) ist neu und ebenfalls Gegenstand der vorliegenden Anmeldung.

Es wird erhalten, wenn man (Verfahren k) 5-Fluor-6-hydroxy-4(1H)-pyrimidinon (XTV) mit einem Chlorierungsmittel, gegebenenfalls in Gegenwart eines Verdünnungsmittels und gegebenenfalls in Gegenwart eines Katalysators, umsetzt.

Das zur Durchführung des erfindungsgemäßen Verfahrens k) als Ausgangsstoff benötigte 5-Fluor-6-hydroxy-4(1H)-pyrimidinon (XTV) ist bekannt und kann nach bekannten Methoden hergestellt werden (JP 61205262; CA: 106:84632).

Die zur Durchführung des erfindungsgemäßen Verfahrens i) als Ausgangsstoffe benötigten Aminophenoxypyrimidine sind durch die Formel (XI) allgemein definiert. In dieser Formel (XI) haben Y¹¹, Y¹², Y¹³, Y¹⁴ und Y¹⁵ vorzugsweise bzw. insbesondere diejenigen Bedeutungen, die bereits im Zusammenhang mit der Beschreibung der Amine der Formel (X) als bevorzugt bzw. als insbesondere bevorzugt für Y¹¹, Y¹², Y¹³, Y¹⁴ und Y¹⁵ angegeben wurden. X² und X⁵ stehen unabhängig voneinander für Chlor oder Fluor.

Die Aminophenoxypyrimidine der Formel (XI) sind neu und ebenfalls Gegenstand der vorliegenden Anmeldung.

Sie werden erhalten, wenn man (Verfahren 1) Trihalogenpyrimidine der Formel (XV), 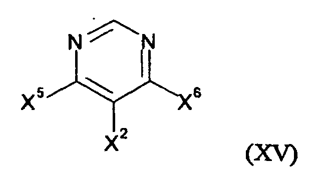
in welcher

- X² und X⁵: die oben angegebenen Bedeutungen haben und
- X⁶: für Fluor oder Chlor steht,

mit einem Aminophenol der Formel (X),
gegebenenfalls in Gegenwart eines Verdünnungsmittels, gegebenenfalls in Gegenwart eines Säureakzeptors und gegebenenfalls in Gegenwart eines Katalysators, umsetzt.

Die zur Durchführung des erfindungsgemäBen Verfahrens 1) als Ausgangsstoffe benötigten Trihalogenpyrimidine sind durch die Formel (XV) allgemein definiert. In dieser Formel (XV) haben X² und X⁵ vorzugsweise bzw. insbesondere diejenigen Bedeutungen, die bereits im Zusammenhang mit der Beschreibung der Verbindungen der Formel (XI) als bevorzugt bzw. als insbesondere bevorzugt für X² und X⁵ angegeben wurden. X⁶ steht für Fluor oder Chlor.

Die Trihalogenpyrimidine der Formel (XV) sind teilweise bekannt und können nach bekannten Methoden hergestellt werden (vergleiche z. B. Chesterfield et al., J. Chem. Soc., 1955; 3478, 3480). Ein Sonderfall der Verbindungen der Formel (XV) ist die erfindungsgemäße Verbindung (XIII); sie kann nach Verfahren k) hergestellt werden.

Die zur Durchführung des erfindungsgemäßen Verfahrens 1) weiterhin als Ausgangsstoffe benötigten Aminophenole der Formel (X) sind bereits weiter oben bei der Beschreibung des erfindungsgemäßen Verfahrens h) beschrieben worden.

Die zur Durchführung des erfindungsgemäßen Verfahrens i) weiterhin als Ausgangsstoffe benötigten Phenole der Formel (XII) sind bereits weiter oben bei der Beschreibung des erfindungsgemäßen Verfahrens j) beschrieben worden.

Die zur Durchführung des erfindungsgemäßen Verfahrens e) als Ausgangsstoffe benötigten O-Benzyl-nitrosalicylsäureamide sind durch die Formel (V), allgemein definiert. In dieser Formel (V) haben Y¹, Y², Y³, Y⁴ und Y⁵ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für Y¹, Y², Y³, Y⁴ und Y⁵ angegeben wurden.

Die O-Benzyl-nitrosalicylsäureamide der Formel (V) sind noch nicht bekannt, sie sind als neue Stoffe Gegenstand der vorliegenden Anmeldung.

Die O-Benzyl-nitrosalicylsäureamide der Formel (V) werden erhalten, wenn man (Verfahren g) O-Benzyl-nitrosalicylsäurederivate der Formel (VI), 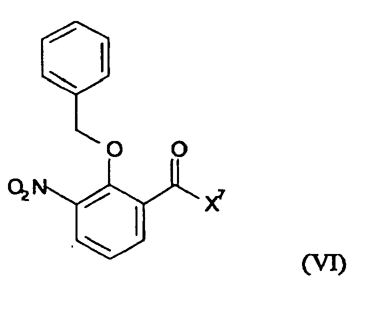
in welcher
- X⁷: für Halogen, Hydroxy oder Alkoxy steht,

mit einem Amin der Formel (VII),
gegebenenfalls in Gegenwart eines Verdünnungsmittels, gegebenenfalls in Gegenwart eines Kondensationsmittel und gegebenenfalls in Gegenwart eines Säureakzeptors, umsetzt.

Die zur Durchführung des erfindungsgemäßen Verfahrens g) als Ausgangsstoffe benötigten O-Benzyl-nitrosalicylsäurederivate sind durch die Formel (VI), allgemein definiert. In dieser Formel (VI) steht X⁷ für Halogen, vorzugsweise Chlor; Hydroxy oder Alkoxy, vorzugsweise Methoxy oder Ethoxy.

Die O-Benzyl-nitrosalicylsäurederivate der Formel (VI) sind bekannt und können nach bekannten Methoden hergestellt werden (vergleiche z. B. J. Am. Chem. Soc. 1959, 5215-5217).

Die zur Durchführung des erfindungsgemäßen Verfahrens g) weiterhin als Ausgangsstoffe benötigten Amine der Formel (VII) bereits weiter oben im Zusammenhang mit der Beschreibung des erfindungsgemäßen Verfahrens f) beschrieben worden.

Die zur Durchführung des erfindungsgemäßen Verfahrens a) weiterhin als Ausgangsstoffe benötigten Acylierungsmittel sind durch die Formel (III), allgemein definiert. In dieser Formel (III) hat R¹ vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R¹ angegeben wurde. X¹ steht für Halogen, Hydroxy, Alkoxy oder Alkylcarbonyloxy, vorzugsweise für Chlor, Hydroxy, Methoxy, Ethoxy oder Acetoxy.

Die Acylierungsmittel der allgemeinen Formel (III) sind bekannte Reagenzien in der organischen Chemie.

Die zur Durchführung des erfindungsgemäßen Verfahrens b) als Ausgangsstoffe benötigten Nitrosalicylsäureamide der Formel (IV) sind bereits weiter oben im Zusammenhang mit der Beschreibung des erfindungsgemäßen Verfahrens d) beschrieben worden.

Die zur Durchführung des erfindungsgemäßen Verfahrens c) als Ausgangsstoffe benötigten O-Benzyl-nitrosalicylsäureamide der Formel (V) sind bereits weiter oben im Zusammenhang mit der Beschreibung des erfindungsgemäßen Verfahrens e) beschrieben worden.

Als Verdünnungsmittel zur Durchführung der erfindungsgemäßen Verfahren a), f) und g) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylforinanilid, N-Methylpyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan.

Als Verdünnungsmittel zur Durchführung der erfindungsgemäßen Verfahren d)und e) kommen alle inerten organischen Lösungsmittel in Betracht. Hierzu gehören vorzugsweise Ester wie Essigsäuremethylester oder Essigsäureethylester; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, Wasser, Salzlösungen, wie beispielsweise Ammoniumchloridlösung, Säuren, wie beispielsweise Salzsäure oder Essigsäure, sowie beliebige Mischungen der genannten Verdünnungsmittel.

Als Verdünnungsmittel zur Durchführung der erfindungsgemäßen Verfahren h), i), j) und I) kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; oder Sulfone, wie Sulfolan.

Als Verdünnungsmittel zur Durchführung des erfindungsgemäßen Verfahrens k) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Nitrile, wie Acetonitril, Propionitril, n- oder i-Butyronitril oder Benzonitril.

Die erfindungsgemäBen Verfahren a), f) und g) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -acetate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b), c), d) und e) werden gegebenenfalls in Gegenwart eines Katalysators durchgeführt. Als solche kommen alle Katalysatoren infrage, die auch für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Das erfindungsgemäße Verfahren c) wird gegebenenfalls auch in Gegenwart von Wasserstoff oder gegebenenfalls in Gegenwart eines unedlen Metalls durchgeführt. Als unedle Metalle seien beispielhaft genannt: Zink, Zinn, Eisen, Aluminium oder Magnesium.

Als weitere Reaktionshilfsmittel zur Durchführung der erfindungsgemäßen Verfahren a), b) und c) kommen alle wasserentziehenden Mittel, insbesondere Essigsäureanhydrid, infrage.

Die erfindungsgemäßen Verfahren f) und g) werden gegebenenfalls in Gegenwart eines Kondensationsmittels durchgeführt. Hierzu gehören vorzugsweise Säurehalogenidbildner wie beispielsweise Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlormethan.

Die erfindungsgemäBen Verfahren h), i), j) und l) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkatimetall- oder Alkalimetallhydride, -hydroxide, -alkoholate, -carbonate oder -hydrogencarbonate, wie beispielsweise Natriumhydrid, Natriumamid, Kalium-tert.-butylat, Natriumhydroxid, Kaliumhydroxid, Natriumcarbonat, Kaliumcarbonat, Kaliumhydrogencarbonat oder Natriumhydrogencarbonat.

Als Katalysatoren für die erfindungsgemäßen Verfahren h), i), j) und l) eignen sich alle Kupfer(I)-Salze, wie beispielsweise Kupfer(I)-chlorid, Kupfer(I)-bromid oder Kupfer(I)-iodid, oder auch Fluoride, wie beispielsweise Natrium-, Kalium- oder Ammoniumfluorid, sowie tertiäre Anunoniumfluoride, wie Tetrabutylammoniumfluorid.

Das erfindungsgemäße Verfahren k) wird gegebenenfalls in Gegenwart eines geeigneten Katalysators durchgeführt. Als solche kommen alle tertiäre Amine infrage, wie beispielsweise Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), sowie Amide, wie Dimethylformamid.

Als Halogenierungsmittel zur Durchführung des erfindungsgemäßen Verfahrens k) kommen alle Reagenzien infrage, die an Kohlenstoff gebundene Hydroxygruppen gegen Chlor austauschen können. Beispielhaft seien genannt: Phosgen, Phosphortrichlorid, Phosphorpentachlorid, Phosphoroxychlorid oder Thionylchlorid und gegebenenfalls zusätzlich Chlorwasserstoff oder Chlor.

Die Reaktionstemperaturen können bei der Durchführung der erfindungsgemäßen Verfahren a), b), c), d), e), f) und g) in einem größeren Bereich variiert werden. Im allgemeinen arbeitet man bei Temperaturen von 0°C bis 180°C, vorzugsweise bei Temperaturen von 0°C bis 130°C.

Die Reaktionstemperaturen können bei der Durchführung der erfindungsgemäßen Verfahren h), i), j) und l) in einem größeren Bereich variiert werden. Im allgemeinen arbeitet man bei Temperaturen von -20°C bis 200°C, vorzugsweise bei Temperaturen von -10°C bis 150°C.

Die Reaktionstemperaturen können bei der Durchführung des erfindungsgemäßen Verfahrens k) in einem größeren Bereich variiert werden. Im allgemeinen arbeitet man bei Temperaturen von 0°C bis 250°C, vorzugsweise bei Temperaturen von 0°C bis 200°C.

Zur Durchführung des erfindungsgemäßen Verfahrens a) zur Herstellung der Verbindungen der Formel (I) setzt man pro Mol des Aminosalicylsäureamids der Formel (II) im allgemeinen 1 bis 2000 Mol, vorzugsweise 1 bis 800 Mol Acylierungsmittel der Formel (III) ein.

Zur Durchführung der erfindungsgemäßen Verfahren b) und c) zur Herstellung der Verbindungen der Formel (I) setzt man pro Mol des Nitrosalicylsäureamides der Formel (IV), bzw. des O-Benzyl-nitrosalicylsäureamides der Formel (V) im allgemeinen 100 bis 2000 Mol, vorzugsweise 200 bis 1000 Mol Ameisensäure ein.

Zur Durchführung des erfindungsgemäßen Verfahrens d) zur Herstellung der Verbindungen der Formel (II) setzt man pro Mol des Nitrosalicylsäureamides der Formel (IV) im allgemeinen 1 bis 100 Mol, vorzugsweise 2 bis 20 Mol Reduktionsmittel ein.

Zur Durchführung des erfindungsgemäßen Verfahrens e) zur Herstellung der Verbindungen der Formel (II) setzt man pro Mol des O-Benzyl-nitrosalicylsäureamides der Formel (V) im allgemeinen 1 bis 100 Mol, vorzugsweise 2 bis 50 Mol Wasserstoff ein.

Zur Durchführung des erfindungsgemäßen Verfahrens f) zur Herstellung der Verbindungen der Formel (IV) setzt man pro Mol Amin der Formel (VII) im allgemeinen 0,5 bis 10 Mol, vorzugsweise 0,8 bis 5 Mol 2-Hydroxy 3-nitrobenzoesäure oder 2-Hydroxy-3-nitrobenzoylchlorid ein.

Zur Durchführung des erfindungsgemäßen Verfahrens g) zur Herstellung der Verbindungen der Formel (V) setzt man pro Mol des Amins der Formel (VII) im allgemeinen 0,5 bis 10 Mol, vorzugsweise 0,8 bis 5 Mol O-Benzylnitrosalicylsäurederivate der Formel (VI) ein.

Zur Durchführung des erfindungsgemäßen Verfahrens h) zur Herstellung der Verbindungen der Formel (VII-a) setzt man pro Mol des Aminophenols der Formel (X) im allgemeinen 0,5 bis 5 Mol, vorzugsweise 0,8 bis 2 Mol Halogenpyrimidin der Formel (IX), bzw. Diazol der Formel (VIII) ein.

Zur Durchführung des erfindungsgemäßen Verfahrens i) zur Herstellung der Verbindungen der Formel (VII-a) setzt man pro Mol des Phenols der Formel (XII) im allgemeinen 0,5 bis 5 Mol, vorzugsweise 0,8 bis 2 Mol Aminophenoxypyrimidin (XI) ein.

Zur Durchführung des erfindungsgemäßen Verfahrens j) zur Herstellung der Verbindungen der Formel (IX-a) setzt man pro Mol des Phenols der Formel (XII) im allgemeinen 0,5 bis 5 Mol, vorzugsweise 0,8 bis 2 Mol 4,6-Dichlor-5-fluorpyrimidin (XIII) ein.

Zur Durchführung des erfindungsgemäßen Verfahrens k) zur Herstellung von 4,6-Dichlor-5-fluorpyrimidin (XIII) setzt man pro Mol 5-Fluor-6-hydroxy-4(1H)-pyrimidinon (XIV) im allgemeinen 0,05 bis 20 Mol, vorzugsweise 0,1 bis 10 Mol Halogenierungsmittel ein.

Zur Durchführung des erfindungsgemäßen Verfahrens 1) zur Herstellung der Verbindungen der Formel (XI) setzt man pro Mol des Aminophenols der Formel (X) im allgemeinen 0,5 bis 5 Mol, vorzugsweise 0,8 bis 2 Mol Trihalogenpyrimidin (XV) ein.

Die erfindungsgemäßen Verfahren a) bis 1) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia- und Phytophtora-Arten, einsetzen. Mit gutem Erfolg werden auch Getreidekrankheiten, wie beispielsweise, Pseudocercosporella-Arten oder Reiskrankheiten, wie beispielsweise Pyricularia-Arten, bekämpft.

Die erfindungsgemäßen Wirkstoffe eignen sich auch zur Steigerung des Ernteertrages. Sie sind außerdem mindertoxisch und weisen eine gute Pflanzenverträglichkeit auf

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder A1kylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natriurn, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazot Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-rnethylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylinethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol.
2-[[6-Deoxy-4-O-(4-O-methyl-β-D-glycopyranosl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl 1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)oxy]-2,5-thiophendicarboxylat,
cis-1-(4-Chlorphenyl)-2-(1H-1,2,4-thiazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Forrnyl-N-hydroxy-DL-alanin-Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cybalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Fiufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

### Herstellungsbeispiele:

### Beispiel (1)

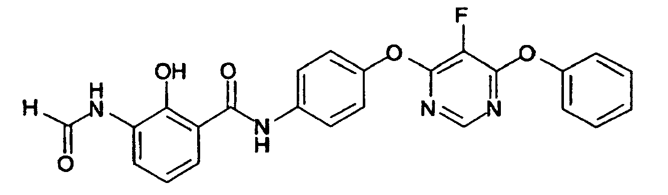

### Verfahren c)

1,67 g (3,02 mMol) 2-Benzyloxy-N-[4-(5-fluoro-6-phenoxy-pyrimidin-4-yloxy)-phenyl]-3-nitro-benzamid werden in 20 ml Ameisensäure mit 0,4 g Raney-Nickel 24 Stunden bei 40°C gerührt. Die Mischung wird auf Wasser gegeben und mehrfach mit Essigsäureethylester extrahiert. Die vereinigten organischen Phasen werden über Natriumsulfat getrocknet und bei vermindertem Druck eingeengt. Der Rückstand wird mit Cyclohexan/Essigester (1:1) an Kieselgel chromatografiert. Man erhält 0,4 g (35 % der Theorie) N-[4-(5-Fluor-6-phenoxy-pyrimidin-4-yloxy)-phenyl]-3-formylamino-2-hydroxy-benzamid als farbloses Öl.
HPLC: logP = 3,31

### Beispiel (1)

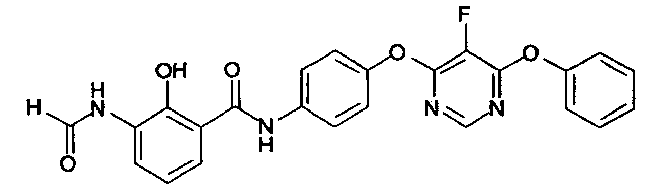

### Verfahren b)

0,5 g (1,08 mMol) N-[4-(5-Fluor-6-phenoxy-pyrimidin-4-yloxy)-phenyl]-2-hydroxy 3-nitrobenzamid werden in 10 ml Ameisensäure mit 0,5 g Raney-Nickel 24 Stunden bei 40°C gerührt. Die Mischung wird auf Wasser gegeben und mehrfach mit Essigsäureethylester extrahiert. Die vereinigten organischen Phasen werden über Natriumsulfat getrocknet und bei vermindertem Druck eingeengt. Der Rückstand wird mit Cyclohexan/Essigester (1:1) an Kieselgel chromatografiert. Man erhält 0,4 g (81 % der Theorie) N-[4-(5-Fluor-6-phenoxy-pyrimidin-4-yloxy)-phenyl]-3-formylamino-2-hydroxy-benzamid als farbloses Öl.
HPLC: logP = 3,31

### Herstellung von Verbindungen der Formel (IV)

### Beispiel (IV-1):

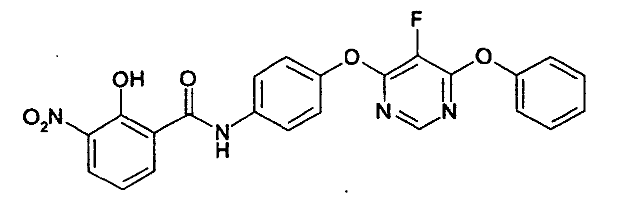

### Verfahren f)

Eine Mischung aus 1,7 g (9 mMol) 3-Nitrosalicylsäure und 2,7 g (9 mMol) 4-(5-Fluor-6-phenoxy-pyrimidin-4-yloxy)-phenylamin in 20 ml Toluol wird unter Rühren auf 50°C erhitzt. Hierzu gibt man 0,5 g Phosphortrichlorid und erhitzt weitere 4 Stunden unter Rückfluß zum Sieden. Nach dem Abkühlen wird das Gemisch eingeengt und der Rückstand mit Dichlormethan extrahiert. Die organische Phase wird mehrfach mit Wasser gewaschen und über Natriumsulfat getrocknet. Nach dem Abdestillieren des Lösungsmittels erhält man 1,7 g (41% der Theorie) N-[4-(5-Fluor-6-phenoxy-pyrimidin-4-yloxy)-phenyl]-2-hydroxy-3-nitrobenzamid.
Massenspektrum: m/e = 462 (M⁺)

### Herstellung von Verbindungen der Formel (V)

### Beispiel (V-1):

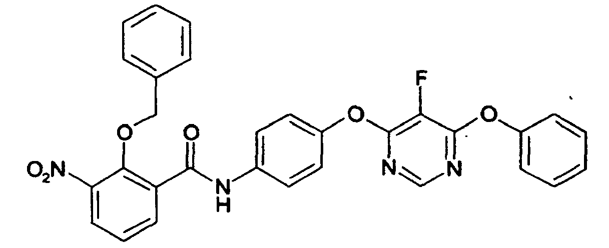

### Verfahren g)

Zu einer Lösung von 4,3 g (18 mMol) 2-Benzyloxy-3-nitro-benzoylchlorid in 20 ml Dichlormethan tropft man bei 0°C eine Lösung von 2,4 g (0,011 Mol) Triethylamin und 5,3 g (18 mMol) 4-(5-Fluor-6-phenoxy-pyrimidin-4-yloxy)-phenylamin in 30 ml Dichlormethan und rührt noch 2 Stunden ohne weitere Kühlung. Die Mischung wird filtriert, das Filtrat mit Wasser gewaschen, über Natriumsulfat getrocknet und bei vermindertem Druck eingeengt. Der Rückstand wird mit Cyclohexan/Essigester (2:1) an Kieselgel chromatografiert. Man erhält 2,8 g (30% der Theorie) 2-Benzyloxy-N-[4-(5-fluoro-6-phenoxy-pyrimidin-4-yloxy)-phenyl]-3-nitro-benzamid als Öl.
HPLC: logP = 4,56

### Herstellung von Verbindungen der Formel (IX-a)

### Beispiel (IX-a-1):

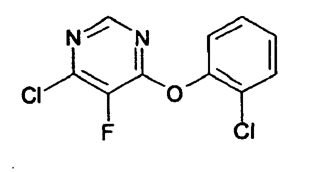

### Verfahren j)

Zu einer Mischung von 5 g (0,0295 Mol) 4,6-Dichlor-5-fluorpyrimidin und 5 g (0,036 Mol) Kaliumcarbonat in 25 ml Acetonitril wird auf 50°C erwärmt. Bei dieser Temperatur tropft man innerhalb von 6 Stunden eine Lösung von 3,8 g (0,03 Mol) 2-Chlorphenol in 25 ml Acetonitril und rührt weitere 6 Stunden bei dieser Temperatur. Nach dem Abkühlen wird das Lösungsmittel bei vermindertem Druck abdestilliert und der Rückstand mit Wasser aufgenommen und mehrmals mit jeweils 40 ml Dichlormethan extrahiert. Die organische Phase wird mit 10%iger Natronlauge gewaschen, über Natriumsulfat getrocknet und wiederum bei vermindertem Druck eingeengt. Man erhält 6,9 g (86,7% der Theorie) 4-Chlor-6-(2-chlorphenoxy)-5-fluor-Pyrimidin.
¹H-NMR: δ = 7,24-7,53 (m, 4H); 8,29 (s, 1H) ppm

### Beispiel (IX-a-2):

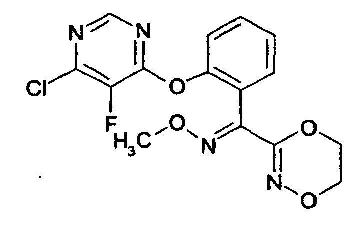

### Verfahren j)

Zu einer Mischung von 3 g (0,018 Mol) 4,6-Dichlor-5-fluorpyrimidin und 2,7 g (0,018 Mol) Kaliumcarbonat in 30 ml Acetonitril wird auf 70 °C erwärmt. Bei dieser Temperatur tropft man innerhalb von 2 Stunden eine Lösung von 3,8 g (0,03 Mol) (5,6-Dihydro-[1,4,2]dioxazin-3-yl)-(2-hydroxy-phenyl)-methanon-O-methyl-oxim in 20 ml Acetonitril und erhitzt weitere 16 Stunden unter Rückfluß. Nach dem Abkühlen wird das Lösungsmittel bei vermindertem Druck abdestilliert und der Rückstand mit Wasser aufgenommen und mehrmals mit jeweils 40 ml Dichlormethan extrahiert. Die organische Phase wird mit 10%iger Natronlauge gewaschen, über Natriumsulfat getrocknet und wiederum bei vermindertem Druck eingeengt. Man erhält 7 g (86,7% der Theorie) [2-(6-Chlor-5-fluorpyrimidin-4-yloxy)-phenyl]-(5,6-dihydro-[1,4,2]dioxazin-3-yl)-methanon-O-methyloxim.
¹H-NMR δ = 3,83 (s, 3H); 4,14 (m, 2H); 4,45 (m, 2H); 7,39-7,54 (m, 4H); 8,32 (s, 1H) ppm

### Herstellung von 4,6-Dichlor-5-fluorpyrimidin

### Beispiel (XIII):

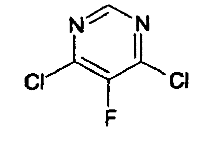

### Verfahren k)

Zu 30 ml Phosphoroxychlorid wird innerhalb von 30 Minuten 7,8 g (0,064 Mol) N,N-Dimethylanilin getropft und 10 Minuten bei 25 °C gerührt. Anschließend gibt man 8,3 g 5-Fluorpyrimidin-4,6-diol zu und erhitzt 15 Stunden unter Rückfluß. Nach dem Abkühlen wird das überschüssige Phosphoroxychlorid im Wasserstrahlvakuum abdestilliert und der Rückstand einer Vakuumdestillation unterworfen. Man erhält 11,2 g (100 % der Theorie) 4,6-Dichlor-5-fluorpyrimidin vom Siedepunkt 58 - 60 °C bei 14 mbar.

### Herstellung von Verbindungen der Formel (VII-a)

### Beispiel (VII-a-1):

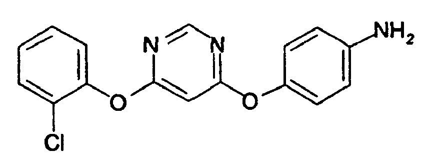

### Verfahren i)

Eine Mischung aus 2 g (9 mMol) 4-(4-Aminophenoxy)-6-chlor-pyrimidin, 1,1 g (9 mMol) 3-Chlorphenol und 2 g (18 mMol) getrocknetes, Kaliumcarbonat in 20 ml Acetonitril wird 4 Stunden unter Rückfluß erhitzt. Anschließend wird die Mischung filtriert und das Filtrat bei vermindertem Druck eingeengt. Der Rückstand wird in Dichlormethan aufgenommen, mit Wasser gewaschen, die organische Phase über Natriumsulfat getrocknet und wiederum bei vermindertem Druck eingeengt. Man erhält 1,3 g (46 % der Theorie) 4-(4-Aminophenoxy)-6-(2-chlorphenoxy)-pyrimidin.
HPLC: logP =1,96

### Herstellung von Verbindungen der Formel (XI)

### Beispiel (XI-1):

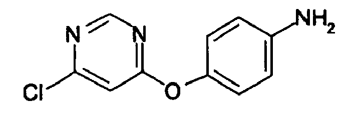

### Verfahren l)

Eine Mischung aus 50 g (0,3 Mol) 4,6-Dichlorpyrimidin, 37 g (0,3 Mol) 4-Aminophenol, 80 g (0,6 Mol) getrocknetes Kaliumcarbonat und 50 mg Kupfer-II-bromid in 600 ml Acetonitril wird 4 Stunden unter Rückfluß erhitzt. Anschließend wird die Mischung filtriert und das Filtrat bei vermindertem Druck eingeengt. Der Rückstand wird in Dichlormethan aufgenommen, mit Wasser gewaschen, die organische Phase über Natriumsulfat getrocknet und wiederum bei vermindertem Druck eingeengt. Man erhält 60 g (58 % der Theorie) 4-(4-Aminophenoxy)-6-chlor-pyrimidin.
HPLC: logP = 0,65

### Beispiel (VII-a-2):

### Verfahren h)

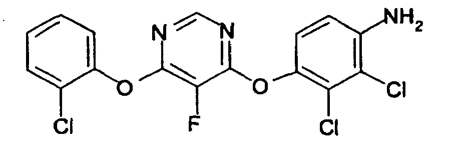

2,7 g 6-Chlor-5-fluor-4-(2-chlorphenyloxy)pyrimidin und 1,5 g Kaliumcarbonat werden in 20 ml Acetonitril vorgelegt und unter RückfluB innerhalb von 1,5 Stunden mit 1,8 g 2,3-Dichlor-4-aminophenol versetzt. Man erhitzt weitere 12 Stunden unter Rückfluß, kühlt ab, entfernt das Lösungsmittel im Vakuum und nimmt den Rückstand in 45 ml Dichlormethan auf. Nach dem Waschen mit 0.25 N wässriger Natronlauge versetzt man die Lösung mit 5 g Aktivkohle, filtriert und destilliert das Lösungsmittel ab. Der Rückstand wird an Kieselgel mit Petrolether/Essigester (2:1) chromatographiert.
Man erhält 2,7 g (64,3 %) an 2,3-DicMor-4-[6-(2-chlorphenoxy)-5-fluorpyrimidin-4-yloxy]-phenylamin vom Schmelzpunkt 162-163°C.

Analog Beispiel 1, sowie entsprechend der allgemeinen Beschreibung der erfindungsgemäßen Verfahren b) und c) wurden die in der nachfolgenden Tabelle 5 genannten Verbindungen der Formel (I-f) erhalten:

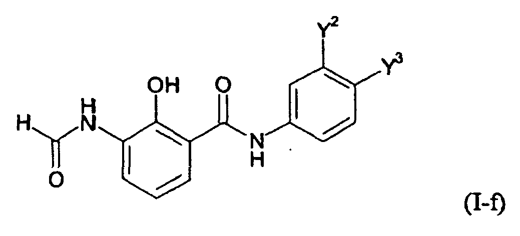

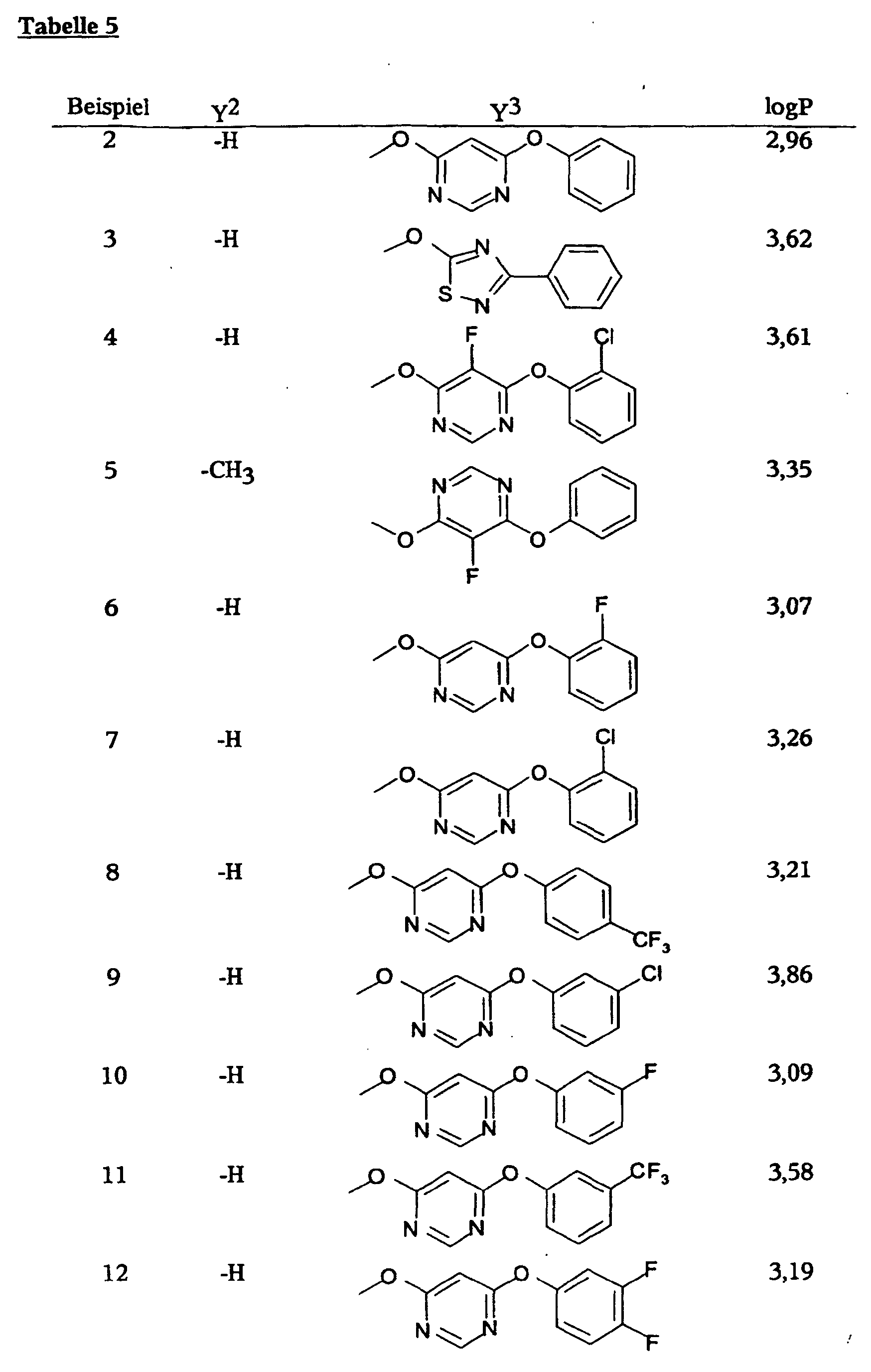

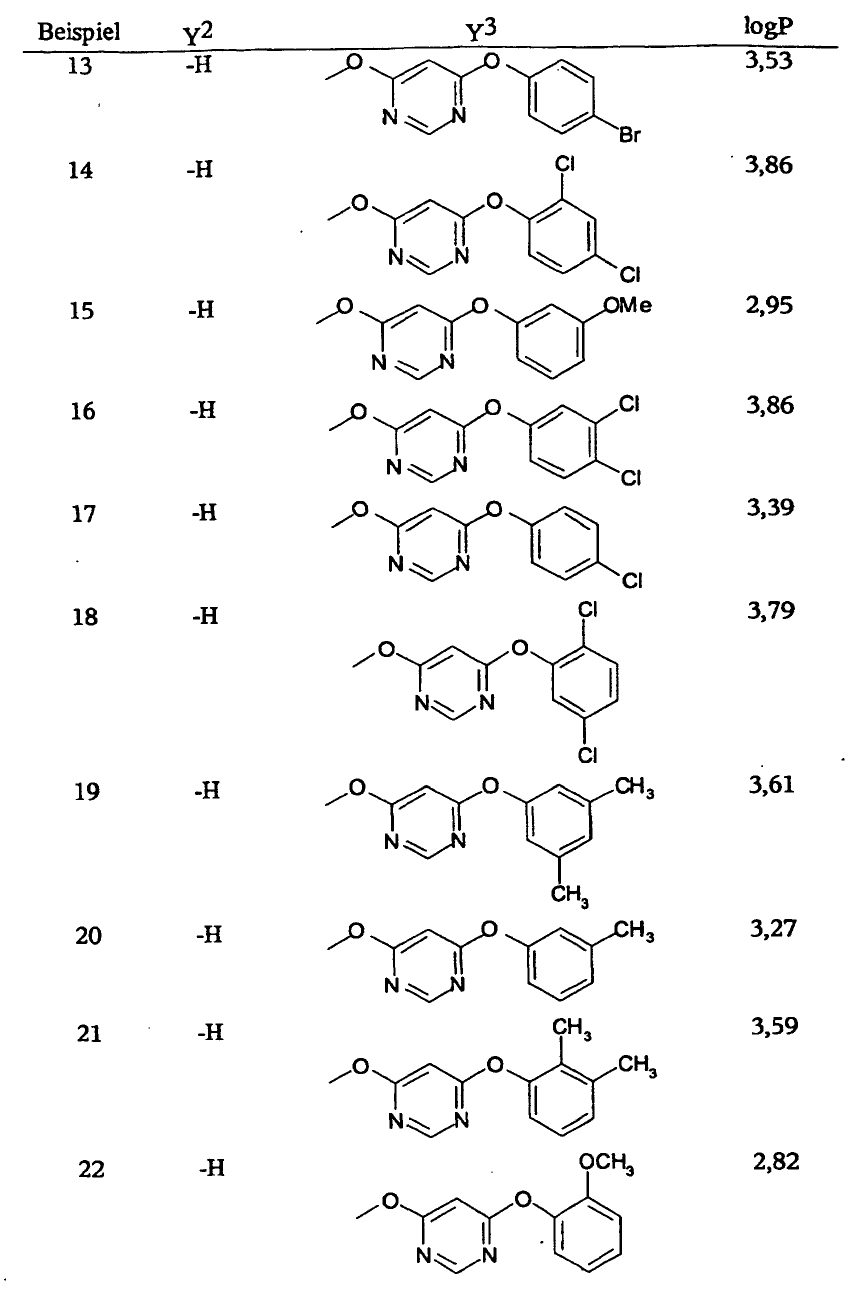

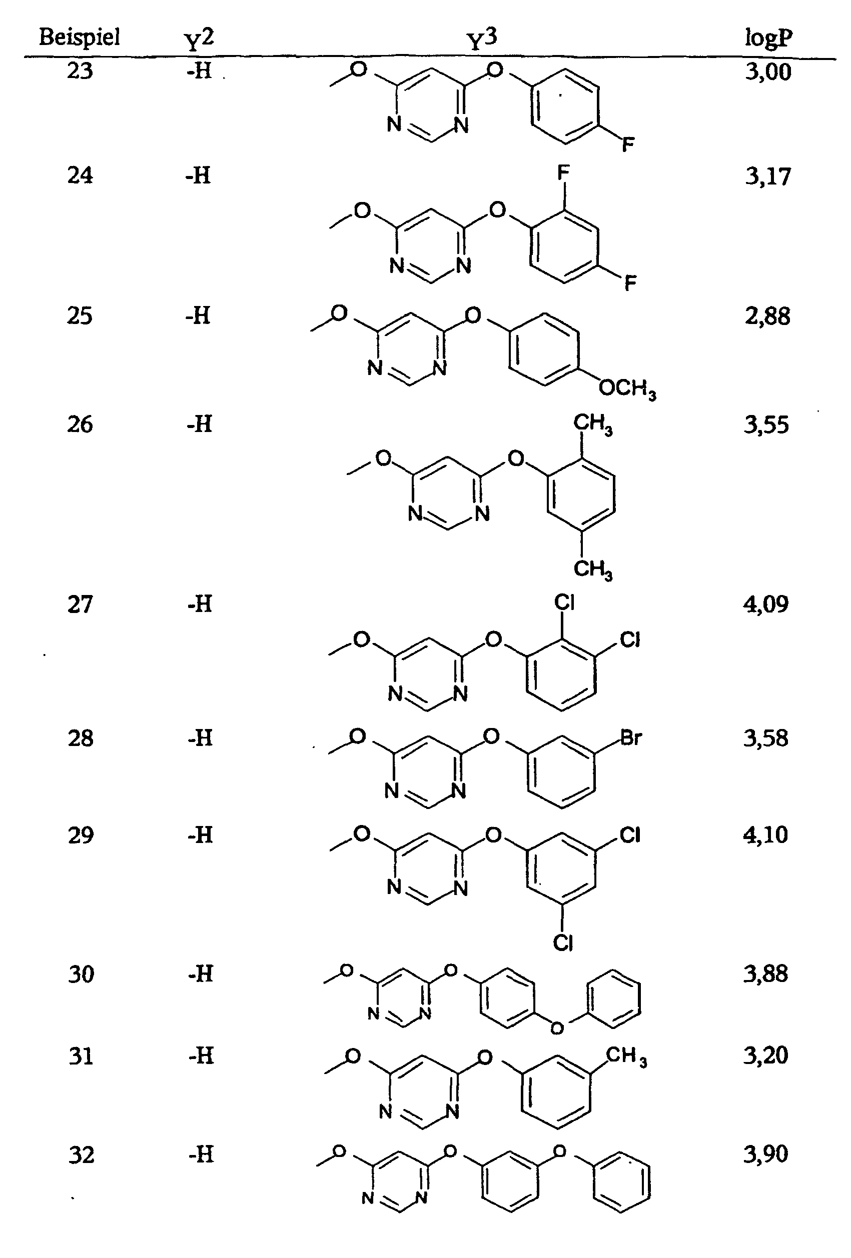

Analog Beispiel (IV-1), sowie entsprechend der allgemeinen Beschreibung des erfindungsgemäßen Verfahrens f) wurden die in der nachfolgenden Tabelle 6 genannten Verbindungen der Formel (IV-a) erhalten:

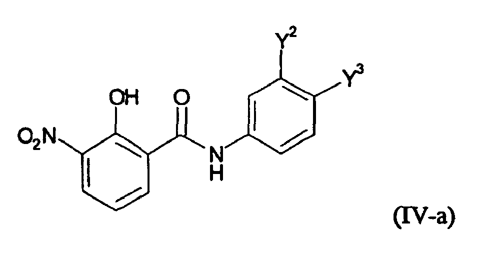

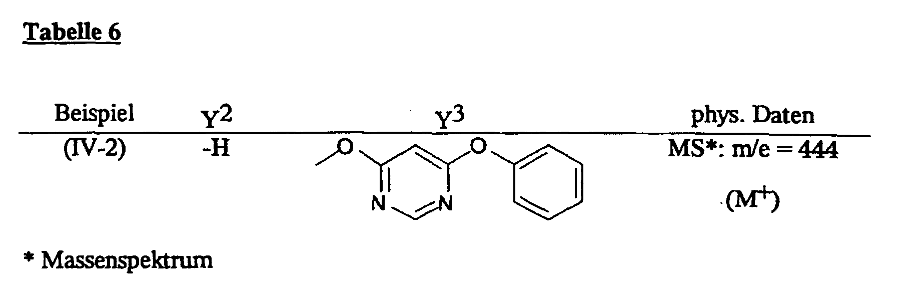

Analog Beispiel (V-1); sowie entsprechend der allgemeinen Beschreibung des erfindungsgemäßen Verfahrens g) wurden die in der nachfolgenden Tabelle 7 genannten Verbindungen der Formel (VI-a) erhalten:

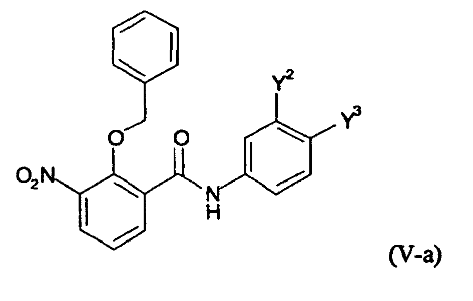

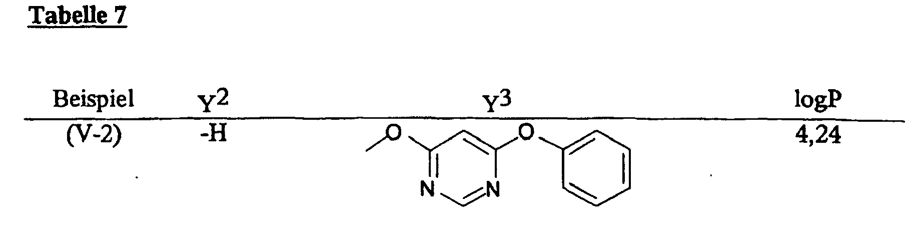

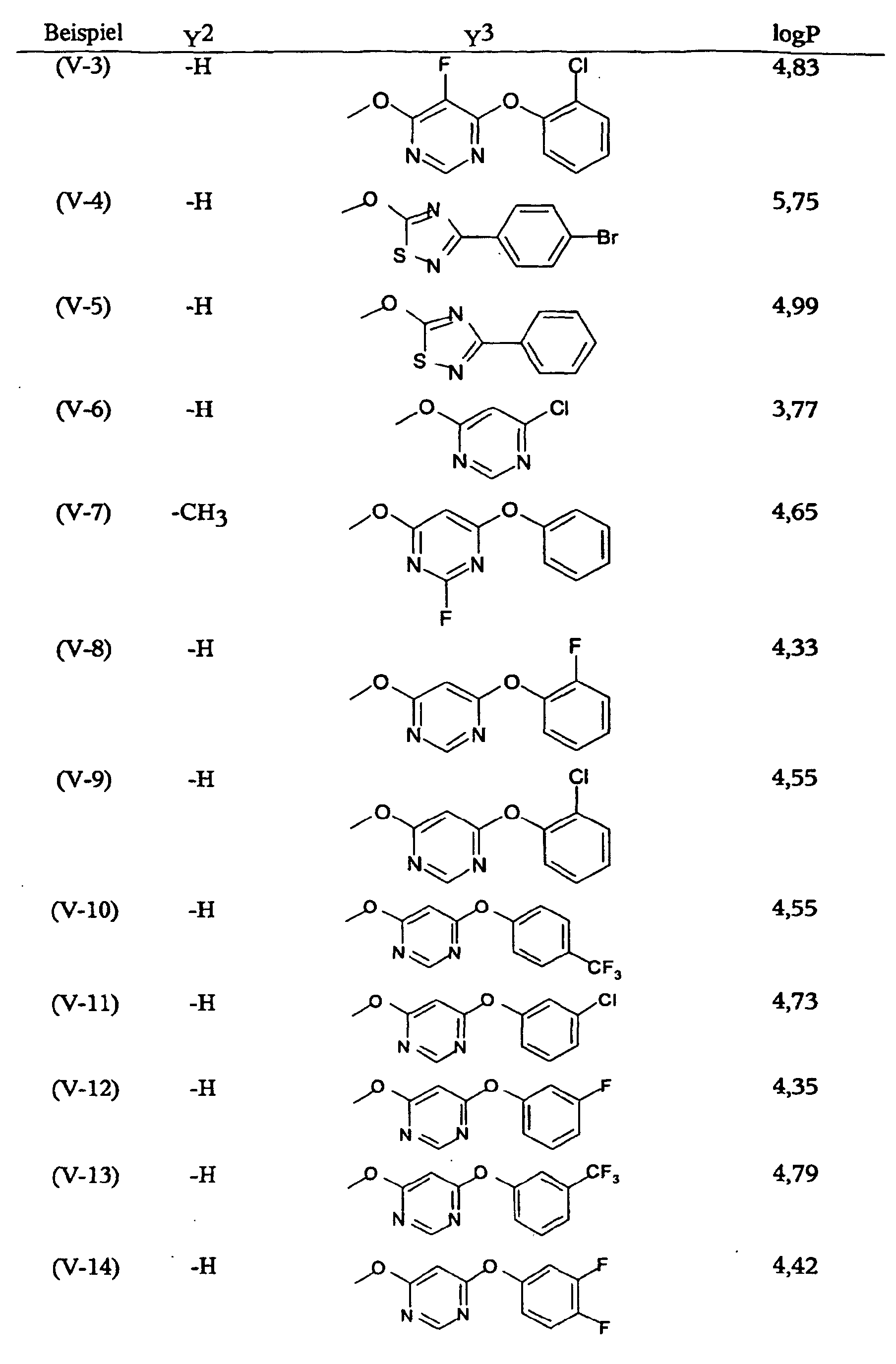

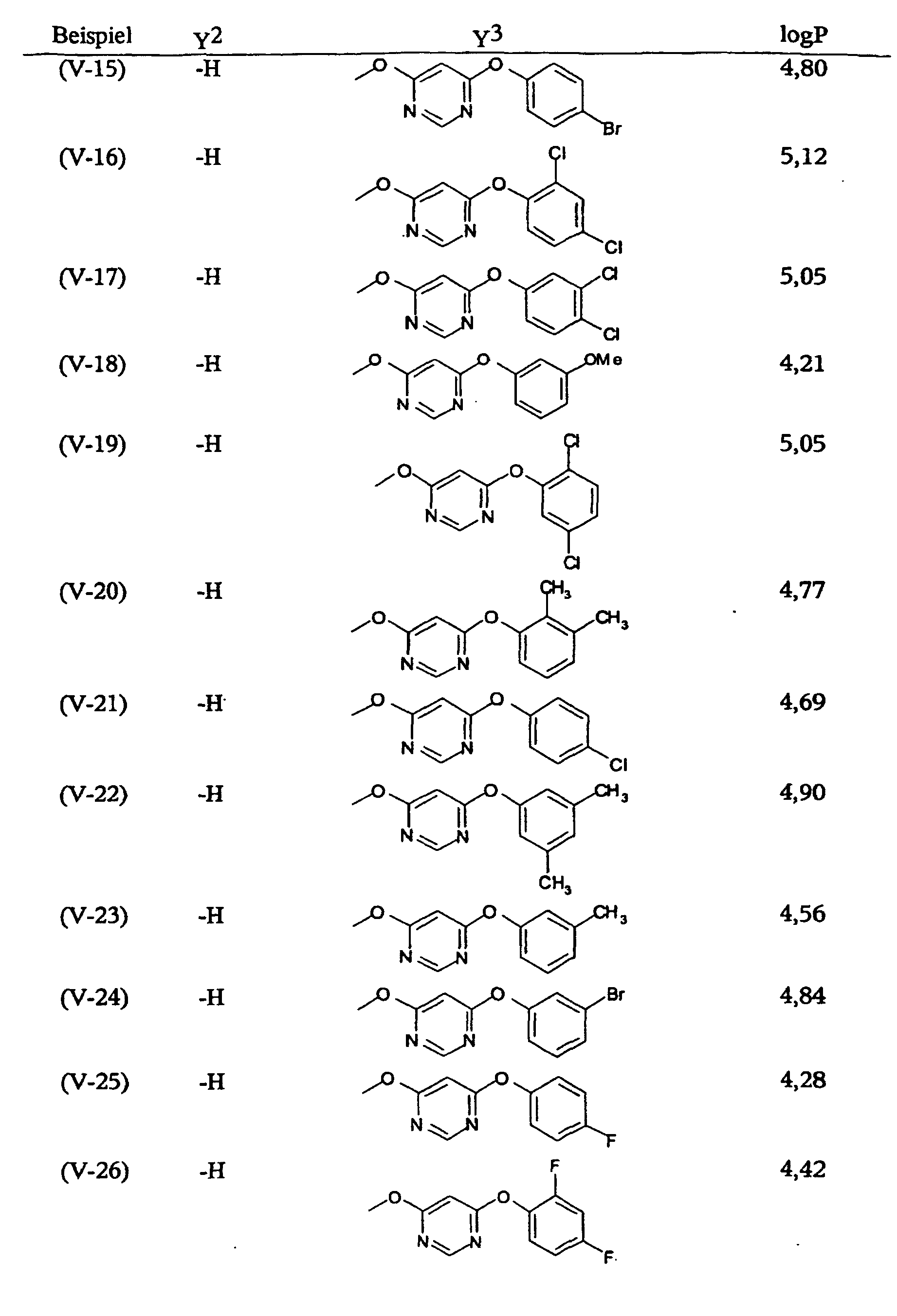

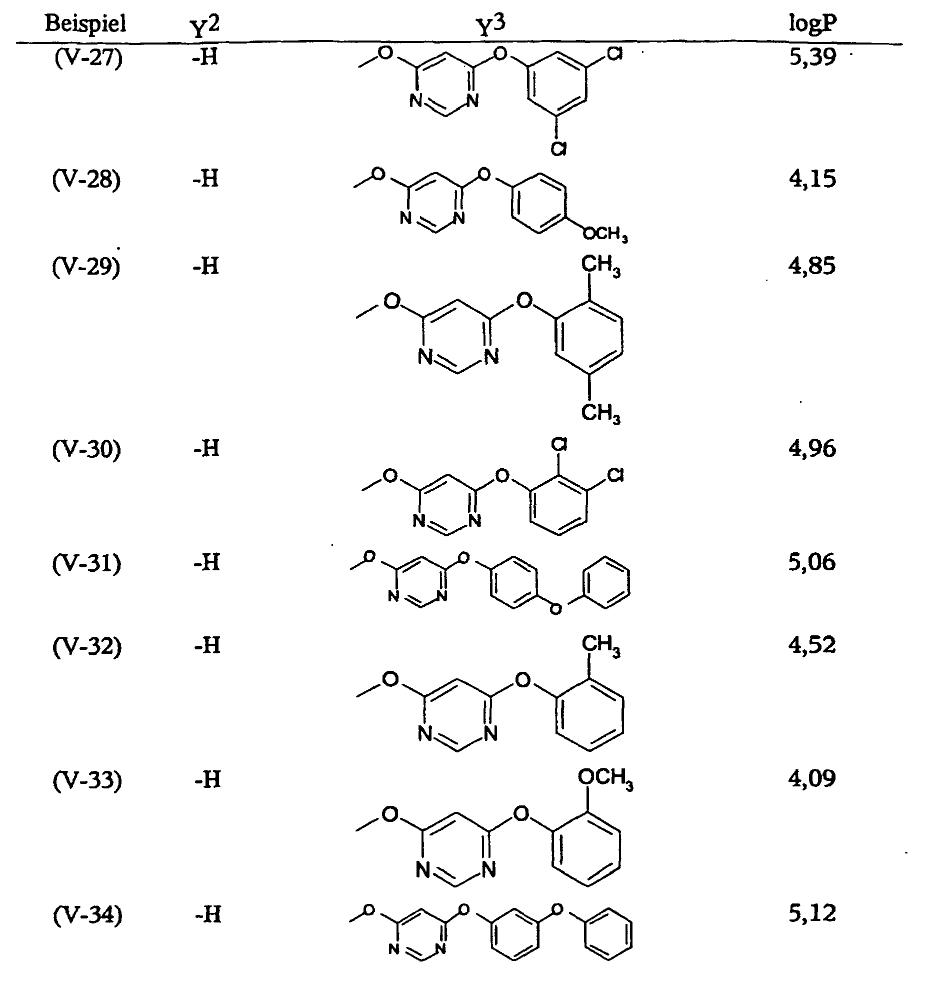

Analog Beispiel (VII-a-1) und (VII-a-2), sowie entsprechend der allgemeinen Beschreibung der erfindungsgemäßen Verfahren h) und i) wurden die in der nachfolgenden Tabelle 8 genannten Verbindungen der Formel (VII-b) erhalten:

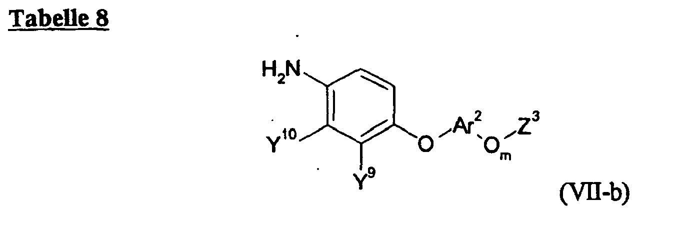

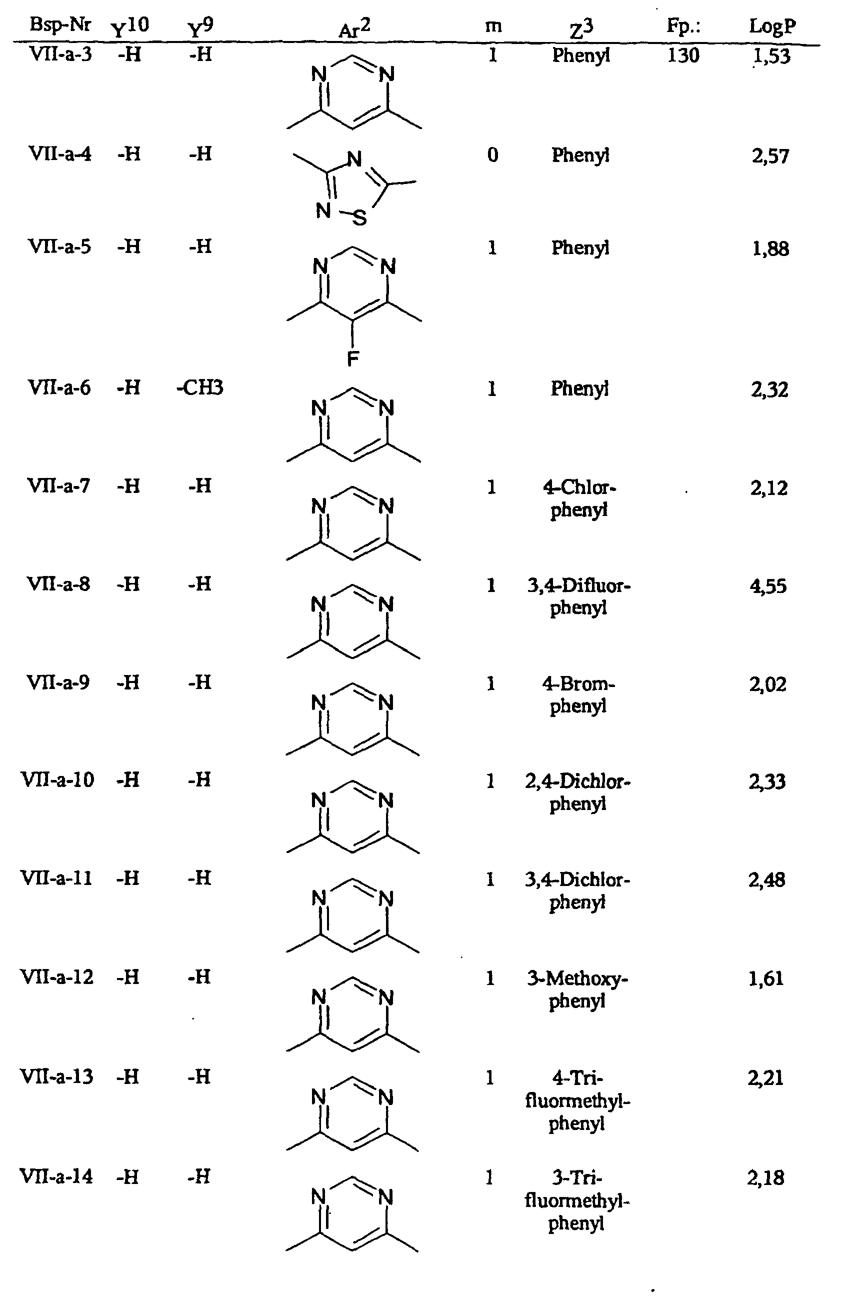

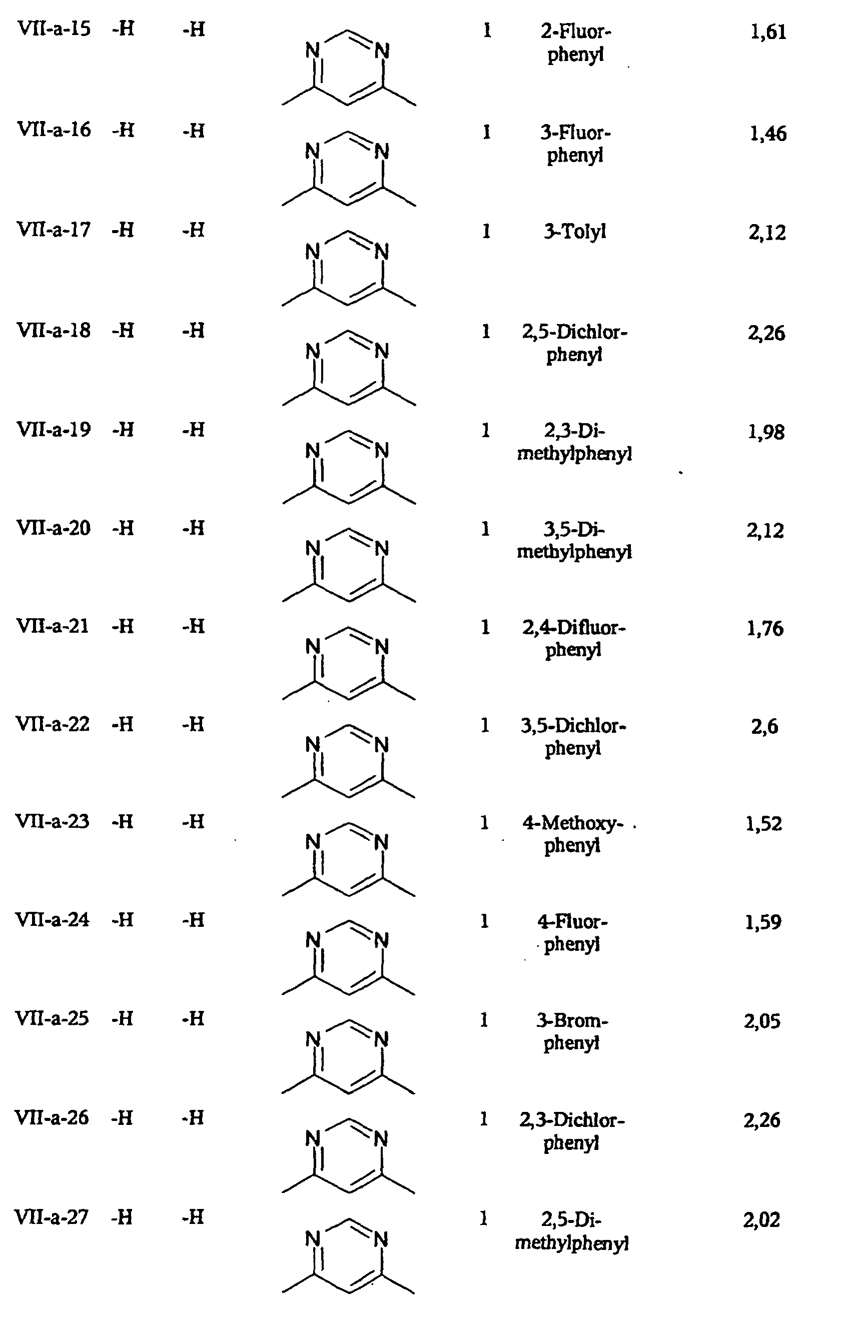

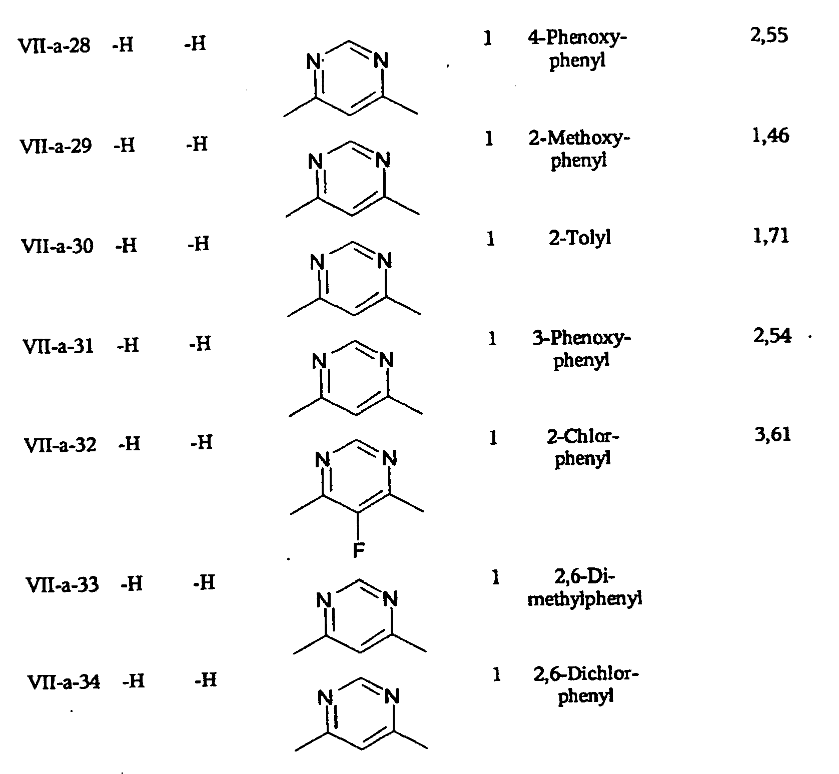

### Anwendungsbeispiele:

### Beispiel A

### Phytophthora-Test (Tomate) / protektiv

| | | |
|---|---|---|
| Lösungsmittel: | 47 | Gewichtsteile Aceton |
| Emulgator: | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt.

3 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1, 2 und 4 bei einer beispielhaften Wirkstoffaufwandmenge von 100 g/ha einen Wirkungsgrad von über 80% im Vergleich zur unbehandelten Kontrolle.

### Beispiel B

### Venturia-Test (Apfel) / protektiv

| | | |
|---|---|---|
| Lösungsmittel: | 47 | Gewichtsteile Aceton |
| Emulgator: | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen z.B. die folgenden Verbindungen der Herstellungsbeispiele 1 und 2 bei einer beispielhaften Wirkstoffaufwandmenge von 100 g/ha einen Wirkungsgrad von über 80% im Vergleich zur unbehandelten Kontrolle.

## Patentansprüche

1. Verbindungen der Formel (I), 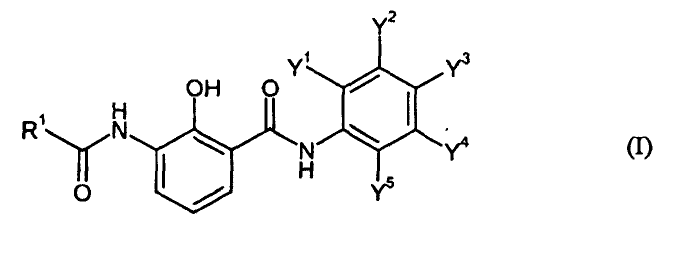
in welcher
R¹ für Wasserstoff, Methyl oder Methoxy steht,
Y¹, Y², Y³, Y⁴ und Y⁵ gleich oder verschieden sind und für Wasserstoff, Fluor, Chlor, Brom, Cyano, Methyl, Ethyl, Methoxy, Trifluormethyl oder Difluormethoxy stehen, und entweder
Y² oder Y³ für -G-Z steht,
worin
G für -T-Ar¹- oder -T-Ar¹-O- steht,
worin
Ar¹ für 1,2,4-Thiadiazoldiyl, 1,3,4-Thiadiazoldiyl, 1,2,4-Oxadiazoldiyl, I,3,4-Oxadiazoldiyl oder für jeweils gegebenenfalls einfach oder zweifach, gleich oder verschieden durch Fluor, Chlor, Cyano, Methyl, Cyclo propyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht und
Z für gegebenenfalls einfach bis dreifach, gleich oder verschieden durch Fluor, Chlor, Cyano oder Methoxy substituiertes Methyl, Ethyl, n- oder i-Propyl, n-, i-, s- oder t-Butyl;
für jeweils gegebenenfalls durch Fluor oder Chlor substituiertes Vinyl, Allyl oder Propargyl;
für jeweils gegebenenfalls einfach bis dreifach, gleich oder verschieden durch Fluor, Chlor, Cyano, Methoxy, Phenyl, Methyl oder Ethyl substituiertes Cyclopropyl, Cyclobutyl, Cyclopentyl oder Cyclohexyl steht; oder
Z für jeweils gegebenenfalls einfach bis dreifach, gleich oder verschieden substituiertes Phenyl, Pyridyl, Pyrimidyl oder Thienyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Di- fluorchlormethoxy, Trifluorethoxy, Difluormethylthio, Trifluormethylthio, Difluorchlormethylthio, Trifluormethylsulfinyl, Trifluormethylsulfonyl, Methoxycarbonyl, Ethoxycarbonyl oder Phenoxy;
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
Cyclopropyl, Cyclobutyl, Cyclopentyl, Cyclohexyl, Phenyl, Phenoxy, oder
eine Gruppierung 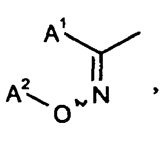
worin
A¹ für Methyl, Ethyl, n- oder i-Propyl, n-, i-, s- oder t-Butyl, Cyclopropyl oder Cyclobutyl steht,
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht, oder
eine Gruppierung 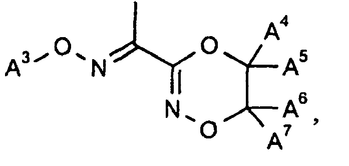
in welcher
A³ für Methyl oder Ethyl steht und
A⁴, A⁵, A⁶ und A⁷ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder
A⁴ und A⁵ oder A⁴ und A⁶ oder A⁶ und A⁷ gemeinsam mit den jeweiligen Kohlenstoffatomen, an die sie gebunden sind, einen cycloaliphatischen Ring mit fünf, sechs oder sieben Kohlenstoffatomen bilden.

2. Verbindungen der Formel (IV), 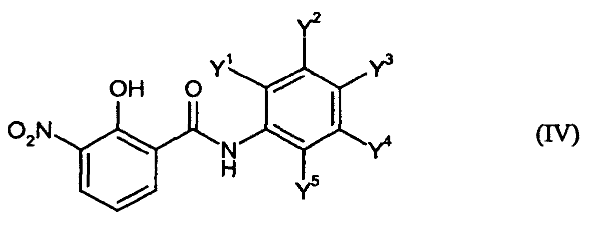
in welcher
Y¹, Y², Y³, Y⁴ und Y⁵ die in Anspruch 1 angegebenen Bedeutungen haben.

3. Schädlingsbekämpfungsmittel, **gekennzeichnet durch** einen Gehalt an mindestens einer Verbindung der Formel (I) und/oder Formel (IV) nach Anspruch 1.

4. Verfahren zur Bekämpfung von Schädlingen, **dadurch gekennzeichnet, dass** man Verbindungen der Formel (I) und/oder Formel (IV) nach Anspruch 1 auf Schädlinge und/oder ihren Lebensraum einwirken lässt.

5. Verfahren zur Herstellung von Verbindungen der Formel (I),
in welcher
R¹, Y¹, Y², Y³, Y⁴ und Y⁵ die in Anspruch 1 angegebenen Bedeutungen haben,
**dadurch gekennzeichnet, dass** man
a) Aminosalicylsäureamide der allgemeinen Formel (II), 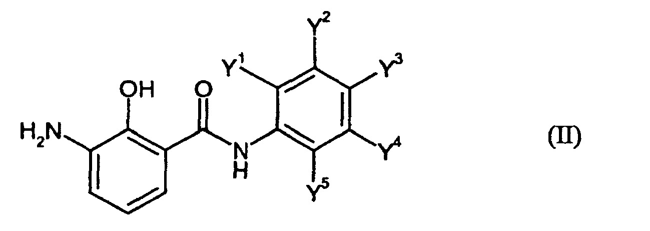
in welcher
Y¹, Y², Y³, Y⁴ und Y⁵ die oben angegebenen Bedeutungen haben,
mit Acylierungsmitteln der allgemeinen Formel (III), 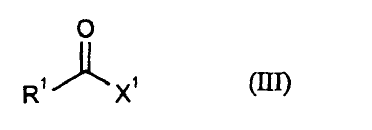
in welcher
R¹ die oben angegebene Bedeutung hat und
X¹ für Halogen, Hydroxy, Alkoxy oder Alkylcarbonyloxy steht,
gegebenenfalls in Gegenwart eines Verdünnungsmittels, gegebenenfalls in Gegenwart eines Säureakzeptors, und gegebenenfalls in Gegenwart eines weiteren Reaktionshilfsmittels, umsetzt, oder
b) Nitrosalicylsäureamide der allgemeinen Formel (IV) 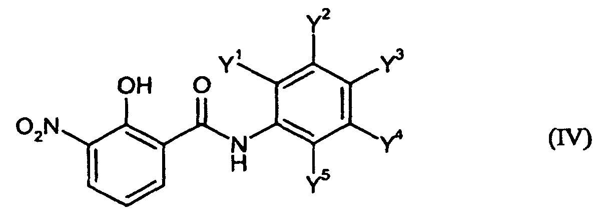
in welcher
Y¹, Y², Y³, Y⁴ und Y⁵ die oben angegebenen Bedeutungen haben,
mit Ameisensäure, gegebenenfalls in Gegenwart eines Katalysators und gegebenenfalls in Gegenwart eines weiteren Reaktionshilfsmittels, umsetzt, oder
c) O-Benzyl-nitrosalicylsäureamide der allgemeinen Formel (V), 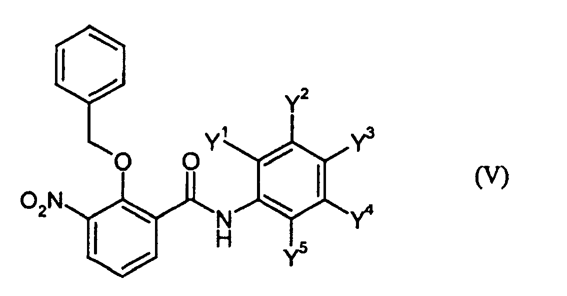
in welcher
Y¹, Y², Y³, Y⁴ und Y⁵ die oben angegebenen Bedeutungen haben,
mit Ameisensäure, gegebenenfalls in Gegenwart von Wasserstoff oder eines unedlen Metalls, gegebenenfalls in Gegenwart eines Katalysators und gegebenenfalls in Gegenwart eines weiteren Reaktionshilfsmittels, umsetzt.

6. Verfahren zur Herstellung von Verbindungen der Formel (IV) wie in Anspruch 2 definiert, **dadurch gekennzeichnet, dass** man (Verfahren f) 2-Hydroxy-3-nitrobenzoesäure oder 2-Hydroxy-3-nitrobenzoylchlorid mit einem Amin der Formel (VII), 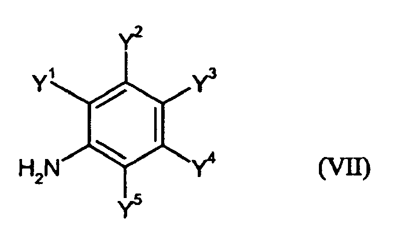
in welcher
Y¹, Y², Y³, Y⁴ und Y⁵ die oben angegebenen Bedeutungen haben,
gegebenenfalls in Gegenwart eines Verdünnungsmittels, gegebenenfalls in Gegenwart eines Kondensationsmittel und gegebenenfalls in Gegenwart eines Säureakzeptors, umsetzt.

7. Verwendung von Verbindungen der Formel (I) und/oder Formel (IV) und Mitteln nach den Ansprüchen 1 bis 2 zur Bekämpfung von Schädlingen.

8. Verfahren zur Herstellung von Schädlingsbekämpfungsmitteln, **dadurch gekennzeichnet, dass** man Verbindungen der Formel (I) und/oder Formel (IV) nach den Ansprüchen 1 bis 2 mit Streckmitteln und/oder oberflächenaktiven Mitteln vermischt.

9. Verbindungen der Formel (II), 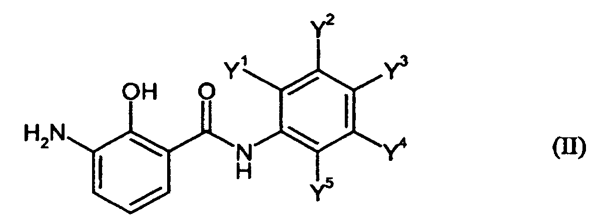
in welcher
Y¹, Y², Y³, Y⁴ und Y⁵ die in Anspruch 1 angegebenen Bedeutungen haben.

10. Verfahren zur Herstellung von Verbindungen der Formel (II) wie in Anspruch 9 definiert, **dadurch gekennzeichnet, dass** man (Verfahren d) Nitrosalicylsäureamide der allgemeinen Formel (IV), 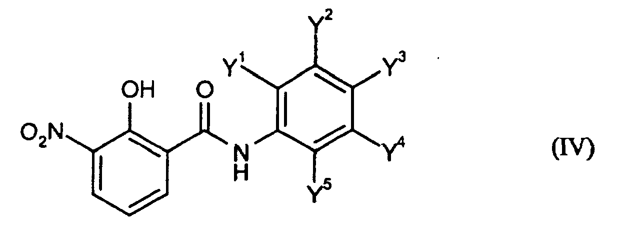
in welcher
Y¹, Y², Y³, Y⁴ und Y⁵ die oben angegebenen Bedeutungen haben,
mit einem Reduktionsmittel, gegebenenfalls in Gegenwart eines Verdünnungsmittels und gegebenenfalls in Gegenwart eines Katalysators, umsetzt,
oder (Verfahren e) O-Benzyl-nitrosalicylsäureamide der allgemeinen Formel (V), 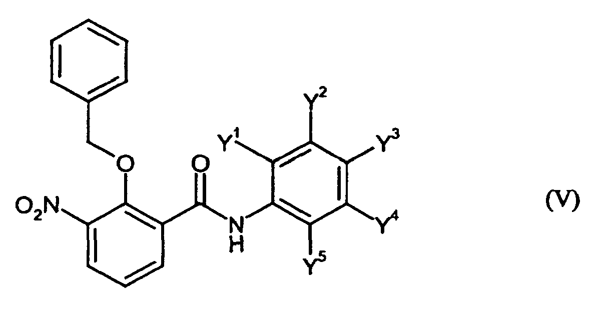
in welcher
Y¹, Y², Y³, Y⁴ und Y⁵ die oben angegebenen Bedeutungen haben,
mit Wasserstoff, gegebenenfalls in Gegenwart eines Verdünnungsmittels, und gegebenenfalls in Gegenwart eines Katalysators, umsetzt.

11. Verbindungen der Formel (V), 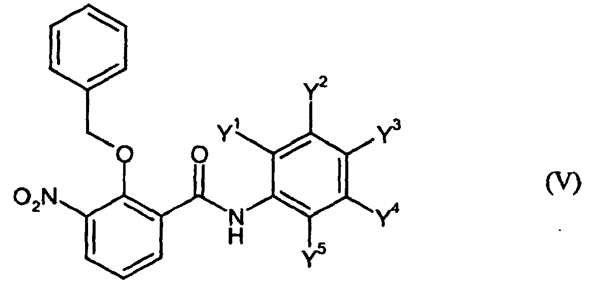
in welcher
Y¹, Y², Y³, Y⁴ und Y⁵ die in Anspruch 1 angegebenen Bedeutungen haben.

## Claims

1. Compounds of the formula (I) 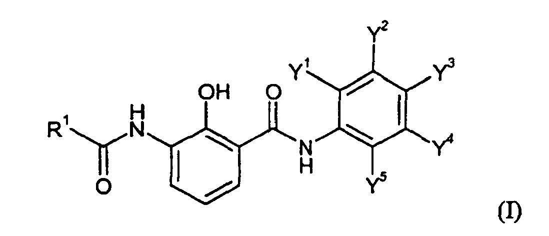
in which
R¹ represents hydrogen, methyl or methoxy,
Y¹, Y², Y³, Y⁴ and Y⁵ are identical or different and each represents hydrogen, fluorine, chlorine, bromine, cyano, methyl, ethyl, methoxy, trifluoromethyl or difluoromethoxy, and either
Y² or Y³ represents -G-Z,
in which
G represents -T-Ar¹- or -T-Ar¹-O-,
in which
Ar¹ represents 1,2,4-thiadiazolediyl, 1,3,4-thiadiazolediyl, 1,2,4-oxadiazolediyl, 1,3,4-oxadiazolediyl or represents pyridinediyl, pyrimidinediyl or 1,3,5-triazinediyl, each of which is optionally mono- or disubstituted by identical or different substituents selected from the group consisting of fluorine, chlorine, cyano, methyl, cyclopropyl, methoxy, methylthio, trifluoromethyl, difluoromethoxy, trifluoromethoxy, difluorochloromethoxy,
T represents a single bond, represents oxygen, sulphur, -CH₂-O-, CH₂-S-, methylene, ethylene or propylene and
Z represents methyl, ethyl, n- or i-propyl, n-, i-, s- or t-butyl, each of which is optionally mono- to trisubstituted by identical or different substituents selected from the group consisting of fluorine, chlorine, cyano or methoxy;
represents in each case optionally fluorine- or chlorine-substituted vinyl, allyl or propargyl;
represents cyclopropyl, cyclobutyl, cyclopentyl or cyclohexyl, each of which is optionally mono- to trisubstituted by identical or different substituents selected from the group consisting of fluorine, chlorine, cyano, methoxy, phenyl, methyl or ethyl; or
Z represents phenyl, pyridyl, pyrimidyl or thienyl, each of which is optionally mono- to trisubstituted by identical or different substituents, where the possible substituents are preferably selected from the list below:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulphinyl, trifluoromethylsulphonyl, methoxycarbonyl, ethoxycarbonyl or phenoxy;
in each case doubly attached methylenedioxy, ethylenedioxy, each of which is optionally mono- to tetrasubstituted by identical or different substituents selected from the group consisting of fluorine, chlorine, methyl, trifluoromethyl and ethyl.
cyclopropyl, cyclobutyl, cyclopentyl, cyclohexyl, phenyl, phenoxy, or
a grouping 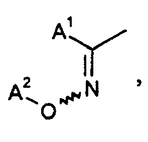
in which
A¹ represents methyl, ethyl, n- or i-propyl, n-, i-, s- or t-butyl, cyclopropyl or cyclobutyl,
A² represents methyl, ethyl, n- or i-propyl, n-, i-, s- or t-butyl, allyl, propargyl, but-2-en-1-yl, 2-methyl-prop- 1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylaminoethyl or benzyl, or
a grouping 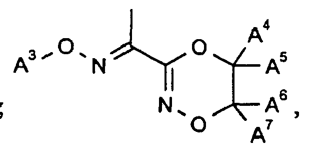
in which
A³ represents methyl or ethyl and
A⁴, A⁵, A⁶ and A⁷ are identical or different and independently of one another each represents hydrogen, methyl, ethyl, n- or i-propyl, n-, i-, s- or t-butyl, hydroxymethyl, trifluoromethyl or trifluoroethyl, or
A⁴ and A⁵ or A⁴ and A⁶ or A⁶ and A⁷ together with the respective carbon atoms to which they are attached form a cycloaliphatic ring having five, six or seven carbon atoms.

2. Compounds of the formula (IV), 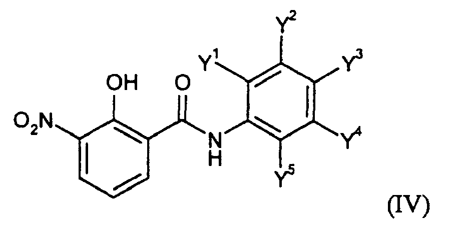
in which
Y¹, Y², Y³, Y⁴ and Y⁵ are each as defined in Claim 1.

3. Pesticides, **characterized in that** they contain at least one compound of the formula (I) and/or formula (IV) according to Claim 1.

4. Method for controlling pests, **characterized in that** compounds of the formula (I) and/or formula (IV) according to Claim 1 are allowed to act on pests and/or their habitat.

5. Process for preparing compounds of the formula (I),
in which
R¹, Y¹, Y², Y³, Y⁴ and Y⁵ are each as defined in Claim 1,
**characterized in that**
a) aminosalicylamides of the general formula (II), 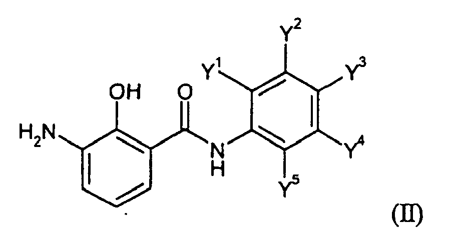
in which
Y¹, Y², Y³, Y⁴ and Y⁵ are each as defined above,
are reacted with acylating agents of the general formula (III), 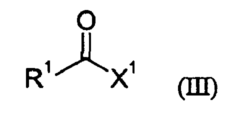
in which
R¹ is as defined above and
X¹ represents halogen, hydroxyl, alkoxy or alkylcarbonyloxy,
if appropriate in the presence of a diluent, if appropriate in the presence of an acid acceptor, and if appropriate in the presence of a further reaction auxiliary, or
b) nitrosalicylamides of the general formula (IV) 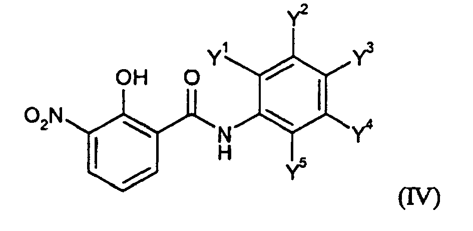
in which
Y¹, Y², Y³, Y⁴ and Y⁵ are each as defined above,
are reacted with formic acid, if appropriate in the presence of a catalyst and if appropriate in the presence of a further reaction auxiliary, or
c) O-benzyl-nitrosalicylamides of the general formula (V), 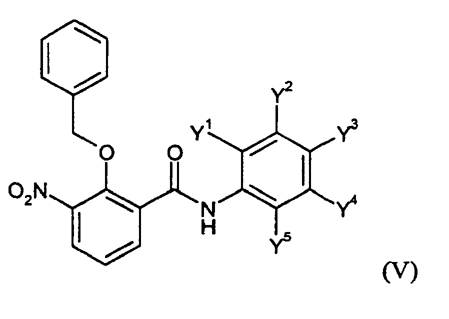
in which
Y¹, Y², Y³, Y⁴ and Y⁵ are each as defined above,
are reacted with formic acid, if appropriate in the presence of hydrogen or a base metal, if appropriate in the presence of a catalyst and if appropriate in the presence of a further reaction auxiliary.

6. Process for preparing compounds of the formula (IV) as defined in Claim 2, **characterized in that** (process f) 2-hydroxy-3-nitrobenzoic acid or 2-hydroxy-3-nitrobenzoyl chloride is reacted with an amine of the formula (VII) 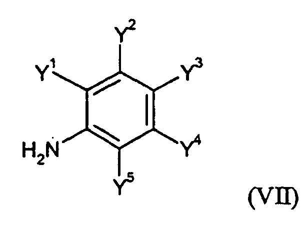
in which
Y¹, Y², Y³, Y⁴ and Y⁵ are each as defined above,
if appropriate in the presence of a diluent, if appropriate in the presence of a condensing agent and if appropriate in the presence of an acid acceptor.

7. Use of compounds of the formula (I) and/or formula (IV) and compositions according to Claims 1 to 2 for controlling pests.

8. Process for preparing pesticides, **characterized in that** compounds of the formula (I) and/or formula (IV) according to Claims 1 to 2 are mixed with extenders and/or surfactants.

9. Compounds of the formula (II) 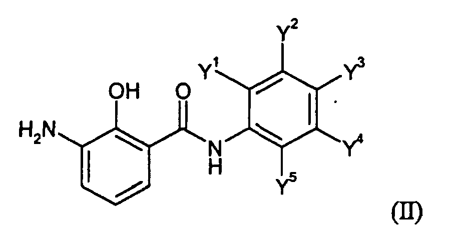
in which
Y¹, Y², Y³, Y⁴ and Y⁵ are each as defined in Claim 1.

10. Process for preparing compounds of the formula (II) as defined in Claim 9, **characterized in that** (process d) nitrosalicylamides of the general formula (IV) 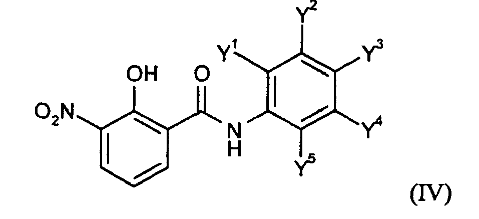
in which
Y¹, Y², Y³, Y⁴ and Y⁵ are each as defined above
are reacted with a reducing agent, if appropriate in the presence of a diluent and if appropriate in the presence of a catalyst,
or (process e) O-benzyl-nitrosalicylamides of the general formula (V) 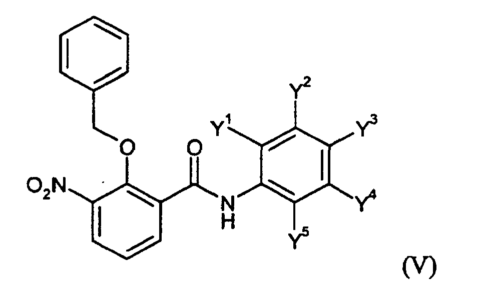
in which
Y¹, Y², Y³ , Y⁴ and Y⁵ are each as defined above
are reacted with hydrogen, if appropriate in the presence of a diluent and if appropriate in the presence of a catalyst.

11. Compounds of the formula (V), 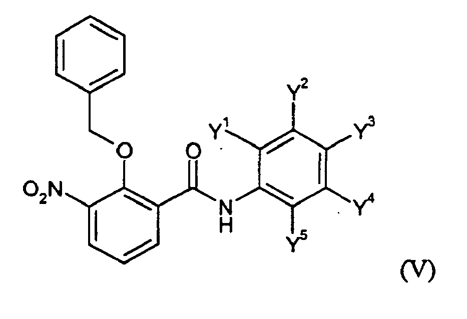
in which
Y¹, Y², Y³, Y⁴ and Y⁵ are each as defined in Claim 1.

## Revendications

1. Composés de formule (I), 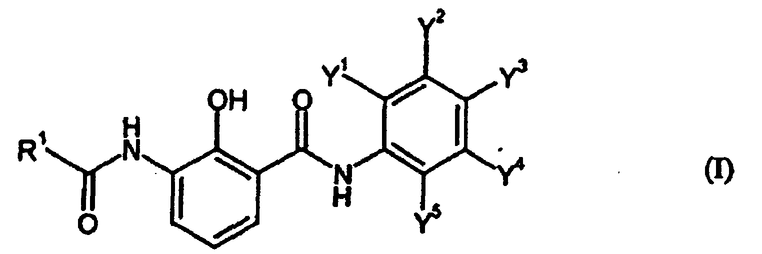
dans laquelle
R¹ représente l'hydrogène, un reste méthyle ou méthoxy,
Y¹, Y², Y³, Y⁴ et Y⁵ sont identiques ou différents et représentent l'hydrogène, le fluor, le chlore, le brome, un groupe cyano, un reste méthyle, éthyle, méthoxy, trifluorométhyle ou difluorométhoxy, et
Y² ou Y³ représente -G-Z,
où
G est un groupe -T-Ar¹- ou -T-Ar¹-O- et,
dans lequel
Ar¹ est un reste 1,2,4-thiadiazolediyle, 1,3,4-thiadiazolediyle, 1,2,4-oxadiazolediyle, 1,3,4-oxadiazolediyle ou représente un reste pyridinediyle, pyrimidinediyle ou 1,3,5-triazinediyle, chacun éventuellement substitué une ou deux fois identiques ou différentes par un radical fluoro, chloro, cyano, méthyle, cyclopropyle, méthoxy, méthylthio, trifluorométhyle, difluorométhoxy, trifluorométhoxy, difluorochlorométhoxy,
T représente une liaison simple, l'oxygène, le soufre, un groupe -CH₂-O-, CH₂-S-, méthylène, éthylène ou propylène et
Z représente un reste éthyle, n-propyle, isopropyle, n-butyle, isobutyle, sec.-butyle, tertiobutyle ou méthyle éventuellement substitué une à trois fois identiques ou différentes par un radical fluoro, chloro, cyano ou méthoxy ;
un reste vinyle, allyle ou propargyle, chacun éventuellement substitué par du fluor ou du chlore ;
un reste cyclopropyle, cyclobutyle, cyclopentyle ou cyclohexyle, chacun éventuellement substitué une à trois fois identiques ou différentes par un radical fluoro, chloro, cyano, méthoxy, phényle, méthyle ou éthyle ; ou bien
Z représente un reste phényle, pyridyle, pyrimidyle ou thiényle, chacun éventuellement substitué une à trois fois identiques ou différentes, les substituants possibles étant avantageusement choisis parmi ceux qui sont énumérés ci-après :
fluor, chlore, brome, cyano, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle ou phénoxy ;
un reste méthylènedioxy, éthylènedioxy, ayant chacun deux liaisons et chacun étant éventuellement substitué une à quatre fois identiques ou différentes par un radical fluoro, chloro, méthyle, trifluorométhyle ou éthyle,
un reste cyclopropyle, cyclobutyle, cyclopentyle, cyclohexyle, phényle, phénoxy, ou
un groupement 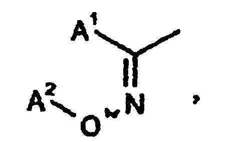
dans lequel
A¹ est un reste méthyle, éthyle, n-propyle, isopropyle, n-butyle, isobutyle, sec.-butyle, tertiobutyle, cyclopropyle ou cyclobutyle,
A² est un reste méthyle, éthyle, n-propyle, isopropyle, n-butyle, isobutyle, sec.-butyle, tertiobutyle, allyle, propargyle, but-2-ène-1-yle, 2-méthyl-prop-1-ène-3-yle, cyanométhyle, méthoxyméthyle, éthoxyméthyle, méthoxyéthyle, éthoxyéthyle, méthylthicanéthyle, éthylthiométhyle, méthylthioéthyle, éthylthioéthyle, diméthylaminométhyle, diméthylaminoéthyle, méthylaminométhyle, méthylaminoéthyle ou benzyle, ou bien
un groupement 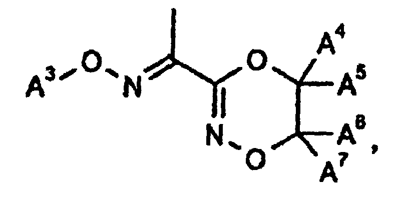
dans lequel
A³ est un reste méthyle ou éthyle et
A⁴, A⁵, A⁶ et A⁷ sont identiques ou différents et représentent indépendamment les uns des autres l'hydrogène, un reste méthyle, éthyle, n-propyle, isopropyle, n-butyle, isobutyle, sec.-butyle, tertiobutyle, hydroxyméthyle, trifluorométhyle ou trifluoréthyle, ou bien
A⁴ et A⁵, ou Aet A⁶, ou A⁶ et A ^{4 7} forment conjointement avec les atomes de carbone correspondants auxquels ils sont liés, un noyau cycloaliphatique ayant cinq, six ou sept atomes de carbone.

2. Composés de formule (IV), 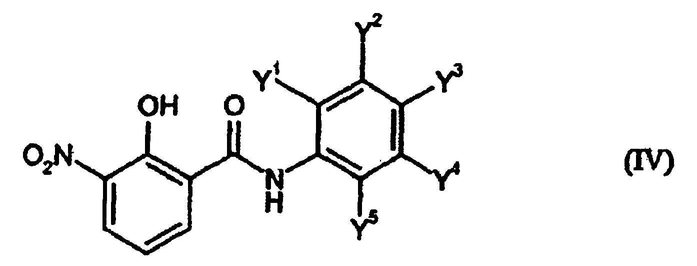
dans laquelle
Y¹, Y², Y³, Y⁴ et Y⁵ ont les définitions indiquées dans la revendication 1.

3. Compositions pesticides, **caractérisées par** une teneur en au moins un composé de formule (I) et/ou de formule (IV) suivant la revendication 1.

4. Procédé pour combattre des parasites, **caractérisé en ce qu'**on fait agir des composés de formule (I) et/ou de formule (IV) suivant la revendication 1 sur les parasites et/ou sur leur milieu.

5. Procédé de production de composés de formule (I), dans laquelle
R¹, Y¹, Y², Y³, Y⁴ et Y⁵ ont les définitions indiquées dans la revendication 1,
**caractérisé en ce que**
a) on fait réagir des amides d'acide aminosalicylique de formule générale (II), 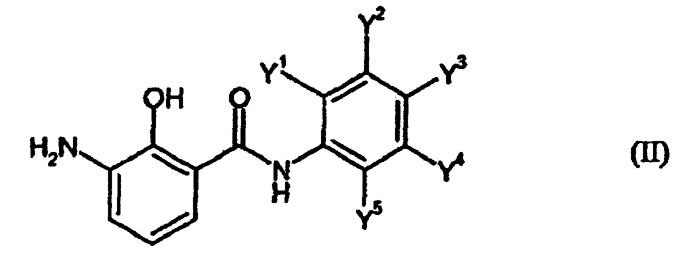
dans laquelle
Y¹, Y², Y³, Y⁴ et Y⁵ ont les définitions indiquées ci-dessus,
avec des agents d'acylation de formule générale (III), 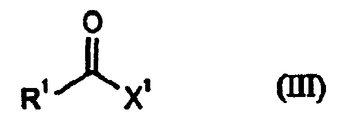
dans laquelle
R¹ a la définition indiquée ci-dessus et
X¹ représente un halogène, un groupe hydroxy, un reste alkoxy ou alkylcarbonyloxy,
éventuellement en présence d'un diluant, le cas échéant en présence d'un accepteur d'acide et, éventuellement, en présence d'un autre auxiliaire de réaction, ou bien
b) on fait réagir des amides d'acide nitrosalicylique de formule générale (IV) 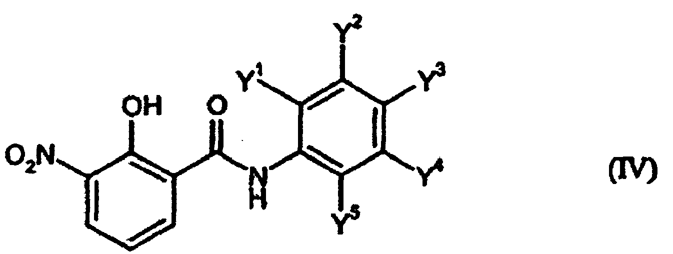
dans laquelle
Y¹, Y², Y³, Y⁴ et Y⁵ ont les définitions indiquées ci-dessus,
avec l'acide formique, éventuellement en présence d'un catalyseur et, le cas échéant, en présence d'un autre auxiliaire de réaction, ou bien
c) on fait réagir des amides d'acide O-benzylnitrosalicylique de formule générale (V), 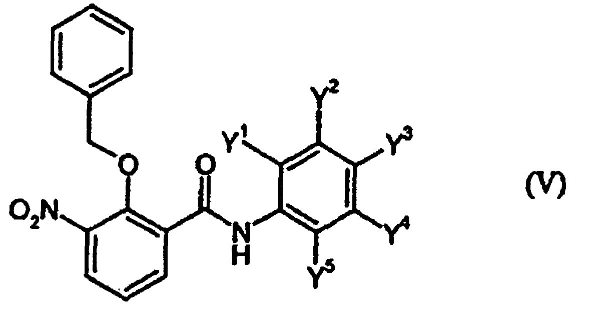
dans laquelle
Y¹, Y², Y³, Y⁴ et Y⁵ ont les définitions indiquées ci-dessus,
avec l'acide formique, éventuellement en présence d'hydrogène ou d'un métal non noble, le cas échéant en présence d'un catalyseur et, éventuellement, en présence d'un autre auxiliaire de réaction.

6. Procédé de production de composés de formule (IV) tels que définis dans la revendication 2, **caractérisé en ce qu'**on fait réagir (procédé f) l'acide 2-hydroxy-3-nitrobenzoïque ou le chlorure de 2-hydroxy-3-nitrobenzoyle avec une amine de formule (VII), 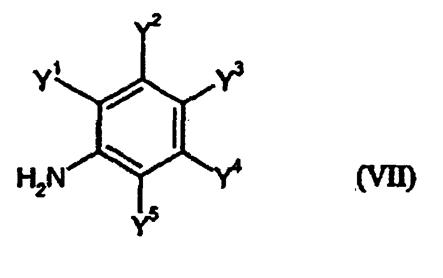
dans laquelle
Y¹, Y², Y³, Y⁴ et Y⁵ ont les définitions indiquées ci-dessus,
le cas échéant, en présence d'un diluant, éventuellement en présence d'un agent de condensation et, le cas échéant, en présence d'un accepteur d'acide.

7. Utilisation de composés de formule (I) et/ou de formule (IV) et de compositions suivant les revendications 1 et 2 pour combattre des parasites.

8. Procédé de préparation de compositions pesticides, **caractérisé en ce qu'**on mélange des composés de formule (I) et/ou de formule (IV) selon les revendications 1 et 2 avec des diluants et/ou des agents tensioactifs.

9. Composés de formule (II), 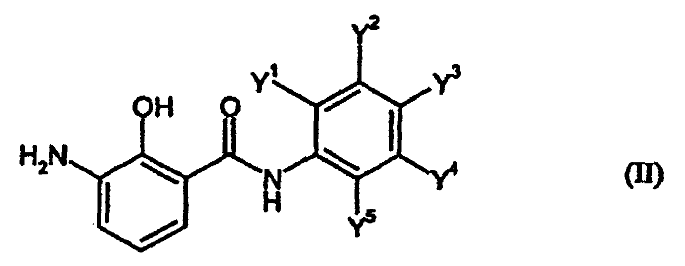
dans laquelle
Y¹, Y², Y³, Y⁴ et Y⁵ ont les définitions indiquées la revendication 1.

10. Procédé de production de composés de formule (II) tels que définis dans la revendication 9, **caractérisé en ce qu'**on fait réagir (procédé d) des amides d'acide nitrosalicylique de formule générale (IV), 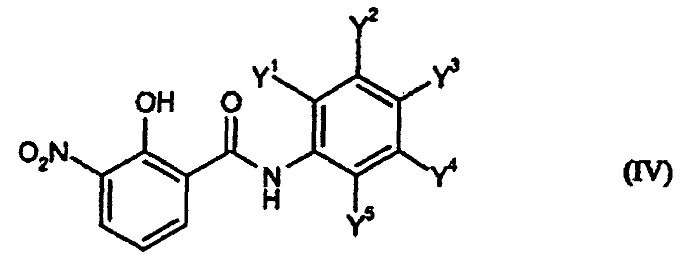
dans laquelle
Y¹, Y², Y³, Y⁴ et Y⁵ ont les définitions indiquées ci-dessus,
avec un agent réducteur, éventuellement en présence d'un diluant et, le cas échéant, en présence d'un catalyseur,
ou bien (procédé e) on fait réagir des amides d'acide O-benzylnitrosalicylique de formule générale (V), 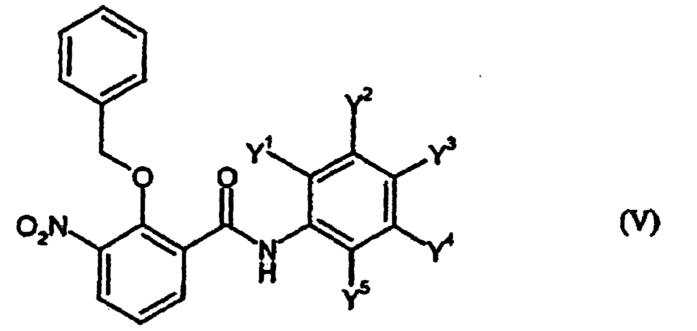
dans laquelle
Y¹, Y², Y³, Y⁴ et Y⁵ ont les définitions indiquées ci-dessus,
avec l'hydrogène, éventuellement en présence d'un diluant et, le cas échéant, en présence d'un catalyseur.

11. Composés de formule (V), 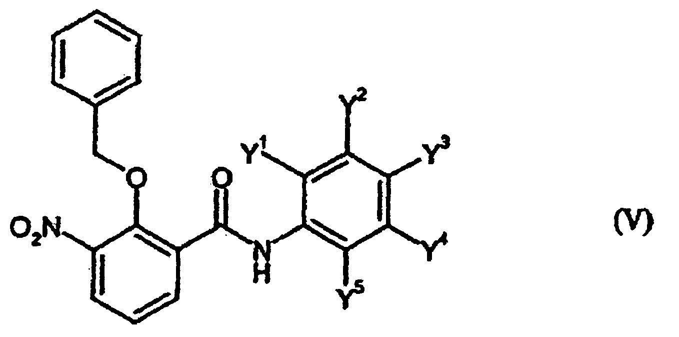
dans laquelle
Y¹, Y², Y³, Y⁴ et Y⁵ ont les définitions indiquées dans la revendication 1.
